# EUROPEAN PATENT SPECIFICATION

(11) **EP 4 199 904 B1**
(45) Date of publication and mention of the grant of the patent: **01.10.2025**
(21) Application number: 21857875.5
(22) Date of filing: 18.08.2021
(51) Int. Cl.: A61K 9/107, A61K 31/352, A61K 47/24, A61K 47/14, A61K 47/12, A61K 31/473, A61K 31/496, A61K 9/48, A61K 31/05

(54) **NANO LIPID CARRIER SYSTEM FOR IMPROVING PERMEATION OF ACTIVE INGREDIENTS**
NANOLIPIDTRÄGERSYSTEM ZUR VERBESSERUNG DER PERMEATION VON WIRKSTOFFEN
SYSTÈME DE SUPPORT NANOLIPIDIQUE PERMETTANT D'AMÉLIORER LA PERMÉATION DE PRINCIPES ACTIFS

(30) Priority: 18.08.2020 IN 202041035519
(43) Date of publication of application: 28.06.2023
(73) Proprietor: Leiutis Pharmaceuticals LLP, Hyderabad 500037 (IN)
(72) Inventor: KOCHERLAKOTA, Chandrashekhar, Telangana Hyderabad 500009 (IN); BANDA, Nagaraju, Telangana Hyderabad 500072 (IN); NARALA, Arjun, Warangal Telangana Hyderabad 506001 (IN); PULLAGURA, Naga Udaya Sankar, Telangana Hyderabad 500054 (IN); AKULA, Srinath, Telangana Hyderabad 500005 (IN)
(74) Representative: Algemeen Octrooi- en Merkenbureau B.V.
(86) International application number: PCT/IB2021/057584
(87) International publication number: WO 2022/038528

(56) References cited:
- WO-A1-2008/144888
- WO-A1-2018/152334
- WO-A1-2020/018512
- WO-A1-2020/123407
- CN-A- 101 439 019
- US-A1- 2018 296 493
- ELMOWAFY MOHAMMED, IBRAHIM HANY M., AHMED MOHAMMED A., SHALABY KHALED, SALAMA AYMAN, HEFESHA HOSSAM: "Atorvastatin-loaded nanostructured lipid carriers (NLCs): strategy to overcome oral delivery drawbacks", DRUG DELIVERY, ACADEMIC PRESS, ORLANDO, FL., US, vol. 24, no. 1, 1 January 2017 (2017-01-01), US , pages 932 - 941, XP055908248, ISSN: 1071-7544, DOI: 10.1080/10717544.2017.1337823
- BHARTI GABA ET AL.: "Nanostructured lipid (NLCs) carriers as a bioavailability enhancement tool for oral administration", DRUG DELIVERY, vol. 22, no. 6, 27 March 2014 (2014-03-27), pages 691 - 700, XP055404310, DOI: 10.3109/10717544.2014.898110

## Description

### FIELD OF THE INVENTION

The present invention relates to a formulation comprising a nano lipid carrier system comprising cannabidiol, low phase transition substance, PEGylated lipid, surfactants and cosolvents, dispersed in water or aqueous medium. The hydrodynamic diameter of the nanocarrier system in aqueous media is less than about 1000nm. The present invention formulation can be in the form of a liquid (solution, suspension, dispersion), liquid filled hard gelatin capsule, soft gelatin capsule or a liquid concentrate.

### BACKGROUND OF THE INVENTION

Nanotechnology has been widely applied in pharmaceuticals for drug delivery. The technology has proven to be useful in formulating delivery systems for small molecules, proteins etc. These improved drug delivery systems can address issues associated with currently used drugs such as increasing efficacy or improving safety and patient compliance. Therefore, nanoparticle systems, such as liposomes, nano-emulsions, and polymeric nanoparticles, have gradually emerged as most investigated systems for drug delivery.

Many therapeutic agents have not been successful because of their limited ability to reach the target tissue. Further, the conventional delivery systems present a lot of disadvantages with regards to safety and efficacy; administration modalities; patient compliance etc.

The advantages of using nanoparticles for drug delivery result from their two main properties. First, nanoparticles, because of their small size, can penetrate through smaller capillaries and are taken up by cells, which allows efficient drug accumulation at the target sites. Second, the use of biodegradable materials for nanoparticle preparation allows window to control the drug release with minimum side effects.

U.S. Pat. No. 4,107,288 describes particles in the size range from 10 to 1,000 nm containing a biologically or pharmacodynamically active material. However, the particles comprise a crosslinked matrix of macromolecules having the active material supported on or incorporated into the matrix.

U.S. Pat. No. 5,145,684 describes a method for providing drug particles having an effective average particle size of less than about 400 nm. The method includes wet milling the drug in the presence of a grinding medium in conjunction with a surface modifier. As in previous methods, the '684 protocol requires grinding or milling to achieve size reduction. The method further requires the use of an additive in the form of a surface modifier.

It is desirable to provide stable dispersible drug particles in nano size range that can be readily prepared using simple and reproducible manufacturing techniques. Moreover, it would be highly desirable to provide pharmaceutical compositions having enhanced permeability.

### SUMMARY OF THE INVENTION

One aspect of the invention is to provide a nano lipid carrier system comprising cannabidiol, one or more low phase transition substance comprising a low phase transition lipid, or mixtures thereof, oil, PEGylated lipid, surfactant and a co-solvent.

Another aspect of the invention is to provide the said nano lipid carrier system wherein the hydrodynamic diameter is less than about 1000nm.

### DETAILED DESCRIPTION OF THE INVENTION

The present invention relates to a novel nano lipid carrier system that improves the oral permeation of cannabidiol. The inventors formulated a novel nano lipid carrier (NLC) system that showed unexpected results in improving permeation rate of active ingredient by more than about five fold.

The NLC system of the present invention comprises (i) one or more low phase transition substance, (ii) oil, (iii) PEGylated lipid (iv) surfactant and (v) a co-solvent. The low phase transition substance may be selected from the group comprising a low phase transition lipid, or mixtures thereof. The inventors surprisingly found that the presence of PEGylated lipid in the NLC formulation increased the permeability of cannabidiol by two to four times.

The NLC system of the present invention has a hydrodynamic diameter (HDD) of less than about 1000nm in aqueous media. Specifically, the system has a hydrodynamic diameter less than about 700nm. More specifically, the hydrodynamic diameter (HDD) of the present invention for NLC formulations have greater than 90% of NLC particles in the range of 100-250 nm and for self-dispersible NLC not less than 70% of self-dispersible NLC particles fall in the range of 250-500 nm. The hydrodynamic diameter can be measured using Particle Size Analyzer (Lite Sizer, Anton Paar). Any of the well-known techniques such as high shear and high pressure homogenization, microfluidization, and membrane emulsification techniques can be used for making the said carrier system.

The novel Nano lipid carrier system can also be formulated as a self-dispersible concentrate comprising of: low phase transition (LPT) substance selected from the group comprising a low phase transition lipid, cannabidiol or mixtures thereof, oil, PEGylated lipid. The system may additionally contain co-solvents, surfactants, stabilizers and optionally organoleptic modifiers. The formulation may be presented as self-dispersible concentrate in liquid or capsule dosage form or as concentrate adsorbed onto a powder matrix. The self-dispersible concentrate when added to aqueous medium forms nanodispersion with minimal agitation.

The NLC system of the present invention comprises
(i) one or more low phase transition substance comprising a low phase transition lipid, or mixtures thereof,
(ii) oil,
(iii) PEGylated lipid
(iv) surfactant and
(v) a co-solvent.

The phase transition temperature herein is defined as the temperature required to induce a change in the lipid physical state from the ordered phase, where the hydrocarbon chains are fully extended and closely packed, to the disordered phase, where the hydrocarbon chains are randomly oriented and fluid. The low phase transition lipid used in this invention is a substance that has a phase transition temperature below 80°C. It may be selected from the group comprising decanoic acid, propyl glycol monostearate (Monosteol), alpha-lipoic acid (ALA), cholesterol, triacylglycerols like tricaprin, trilaurin, trimyristin, tripalmitin and tristearin, acylglycerols like glycerol monostearate (Imwittor 900), glycerol behenate (Compritol 888 ATO) and glycerol palmitostearate (Precirol ATO 5), fatty acids like stearic acid, palmitic acid and myristic acid, lauric acid, waxes like carnauba wax, cetyl palmitate and the like. The lipid may not be limited to the excipients described herein and may be a mixture of one or more lipids. Decanoic acid, Monosteol, alpha lipoic acid, cholesterol are preferred. The low phase transition substance may also be cannabidiol and its derivatives.

Cannabidiol has the following structure and is chemically 2-[(1R,6R)-3-Methyl-6-(1-methylethenyl)-2cyclohexen- 1-yl]-5-pentyl-1,3-benzenediol Its empirical formula is C₂₁H₃₀O₂

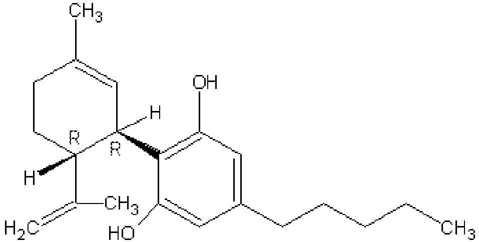

Cannabidiol is approved in the US as Epidiolex for the treatment of seizures associated with Lennox-Gastaut syndrome, Dravet syndrome, or Tuberous sclerosis complex in patients 1 year of age and older.

**Table 1: List of low phase transition lipids**

| **S. No** | **Name of Lipids** | **Transition temperature** |
|---|---|---|
| 1. | decanoic acid | 31.2-33.2°C |
| 2. | alpha lipoic acid | 60-62°C |
| 3. | Monosteol (propylene glycol monostearate) | 35-38.5°C |
| 4. | Precirol ATO 5 (glyceryl palmitostearate) | 50-60°C |
| 5. | Imwittor 900 (glycerol monostearate) | 55-60°C |
| 6. | Compritol 888 ATO (glycerol behenate) | 65-77°C |
| 7. | lauric acid | 44-46°C |
| 8. | myristic acid | 52-54°C |
| 9. | stearic acid | 67-72°C |
| 10. | palmitic acid | 61-62.5°C |
| 11. | Dynasan 114 (glyceryl trimyristate) | 56-57°C |
| 12. | Dynasan 116 (glyceryl tripalmitate) | 66-67°C |
| 13. | Gelucire 50/13 (stearoyl polyoxylglycerides) | 46-51°C |

The low phase transition substance is a lipid, it may be present upto 20%, more preferably upto 15% by weight of the composition. Cannabidiol may be present upto 35%, preferably upto 30% by weight of the composition.

The oil can be selected from the group comprising medium chain triglycerides such as Labrafac Liphophile WL 1349 (Medium chain Triglycerides NF), caprylic acid and the like, fish Oil (Incromega), soyabean oil, olive oil, corn oil, vitamin E, grapeseed oil, walnut oil, avocado oil, flax seed oil, coconut oil, olive oil, hemp seed oil, ginger oil, eicosapentaenoic acid (EPA), docosahexaenoic acid (DHA), omega fatty acid (OFA) and the like. Medium chain triglycerides, fish Oil, omega fatty acid are preferred. The oil may constitute upto 50% by weight of the composition or more preferably upto 45% by weight of the composition.

PEGylated lipid herein shall mean a Lipid molecule with attached PEG wherein molecular weight of PEG ranges from 200Da to 20000 Da. The class of lipids encompasses fatty acids, sterols, glycerol derivatives of fatty acids, monoglycerides, diglycerides, triglycerides, hydrophobic or amphiphilic molecules, waxes, phospholipids, sterols, vitamins, oils, cholesterol, and of the kind. More specifically, the PEGylated lipids can be chosen from the group comprising N-(Carbonyl-methoxypolyethylenglycol-2000)-1,2-distearoyl-sn-glycero-3-phosphoethanolamine, MPEG-2000-DSPE, Na-salt (LIPOID PE 18:0/18:0-PEG 2000), Polyethylene glycol-15-hydroxystearate (Solutol HS-15), N-(Carbonyl- methoxypolyethylenglycol-2000)-1,2-dimyristoyl-sn-glycero-3 phosphoethanolamine, MPEG-2000-DMPE, sodium salt (LIPOID PE 14:0/14:0-PEG 2000), N-(Carbonyl-methoxypolyethylenglycol-5000)-1,2-dimyristoyl-sn-glycero-3-phosphoethanolamine, MPEG-5000-DMPE, sodium salt (LIPOID PE 14:0/14:0-PEG 5000), N-(Carbonyl-methoxypolyethylenglycol-2000)-1,2-dipalmitoyl-sn-glycero-3-phosphoethanolamine, MPEG-2000-DPPE, sodium salt (LIPOID PE 16:0/16:0-PEG 2000), N-(Carbonyl-methoxypolyethylenglycol-5000)-1,2-dipalmitoyl-sn-glycero-3-phosphoethanolamine, MPEG-5000-DPPE, sodium salt (LIPOID PE 16:0/16:0-PEG 5000), N-(Carbonyl-methoxypolyethylenglycol-5000)-1,2-distearoyl-sn-glycero-3-phospho ethanolamine, MPEG-2000-DSPE, Na-salt (LIPOID PE 18:0/18:0-PEG 5000) (PEG- PE), Polyethylene glycol monostearate (Gelucire^{®} 48/16).

The most preferred PEGylated lipid is PEG-PE. The inventors surprisingly found that permeability of cannabidiol (CBD) is increased in the NLC system by about 2 fold in presence of PEGylated lipid than compared to corresponding preparation without PEGylated lipid. This data is presented in table 6. Further, the inventors also surprisingly found that permeability of active ingredient increased by about 4 times in NLC that contained combination of cannabidiol and PEGylated lipid than compared to NLC formulation of active ingredient that did not contain cannabidiol and PEGylated lipid. The PEGylated lipids, preferably PEG-PE, may be present upto 10% by weight of the composition.

The co solvents can be selected from the group comprising ethanol, N-methyl-2-pyrrolidone (NMP), tertiary butyl alcohol (TBA), glycerol, propylene glycol, polyethylene glycol, Transcutol HP, Gelucire^{®} 50/13 (stearoyl polyoxylglycerides). The cosolvents may be present upto 10% by weight of the composition.

Surfactants can be selected from the group comprising amphiphilic surfactants, lipophilic surfactants comprising like alpha lecithin, cholesterol oleate; phospholipids like soy lecithin, egg lecithin (LIPOID E 80 S), hydrogenated soy lecithin, alpha lecithin; modified phospholipids derivatives of dimyristoyl phosphatidylcholine (DMPC), 1,2-dipalmitoyl-rac-glycero-3-phosphocholine (DPPC), dimyristoyl phosphatidyl ethanolamine (DMPE), 1,2-bis(diphenylphosphino) ethane (DPPE), dimyristoylphosphatidylglycerol (DMPG), dipalmitoyl phosphatidylglycerol (DPPG), 1,2-distearoyl-sn-glycero-3-phosphorylethanolamine (DSPE), 1,2-dioleoyl-sn-glycero-3-phosphoethanolamine (DOPE), hydrophilic surfactants like poloxamer-188, sodium oleate, sodium cholate, ethylene oxide/propylene oxide copolymers like poloxamer 182, poloxamer 407 and poloxamine 908, poloxamer-188, non-ionic surfactants like polysorbate 20, polysorbate 60, polysorbate 80 (Tween 80), Span-20, Span-60 and Span-80, cremophor EL, anionic bile salts sodium cholate, sodium glycocholate, sodium tauro cholate, sodium tauro deoxy cholate and sucrose esters of fatty acids (ex: sucrose laurate, sucrose oleate, sucrose palmitate, sucrose stearate and the like), Kolliphor RH40, as aqueous solution or dispersion of surfactants. The quantity of surfactants may be upto 10% by weight of the composition.

The stabilizers may be selected from antioxidants like vitamin E acetate, ascorbyl palmitate, ascorbic acid, monothioglycerol (MTG), citric acid, tartaric acid etc, chelating agents like 1,4,7,10-tetraazacyclododecane-1,4,7,10-tetraacetic acid (DOTA), disodium EDTA, preservatives like methyl paraben, propyl paraben, chlorbutanol and the like.

Buffers may be selected from the group comprising L-histidine, L-arginine, tris base, meglumine, glycine, sodium succinate, diethanol amine, aspartic acid, glutamic acid, alanine, sodium acetate, sodium ascorbate, sodium citrate, citric acid, succinic acid, triethanolamine, boric acid, sodium hydroxide, sodium bicarbonate etc. L-histidine, L-arginine, tris base, meglumine, glycine are preferred.

The NLC system may also contain tonicity modifiers like dextrose, mannitol, glycerol, sodium chloride, organoleptic additives like sucralose, aspartame, saccharin, neohesperidine dihydrochalcone, neotame, alitame, erythritol flavouring agents like peppermint supreme, masking agent 2522, pine apple flavour, lemon flavour, lime flavor, masking agent 2521, menthol, spearmint oil, cinnamon oil.

In another embodiment, the NLC system is used to formulate a combination of cannabidiol with netupitant, palonosetron, atorvastatin, gabapentin, naproxen, abiraterone, cabazitaxel, paclitaxel, duloxetine and many other active ingredients and combinations thereof.

Netupitant is 2-[3,5-bis(trifluoromethyl)phenyl]-N,2-dimethyl-N-[4-(2 methylphenyl)-6-(4-methylpiperazin-1-yl)pyridin-3-yl]propenamide. Netupitant in combination with Palonosetron is commercially marketed as Akynezo as a capsule. The absorption of Netupitant from Akynezo is reported to be in range of 63% to 87% with Tₘₐₓ of about 5hrs in healthy subjects. Netupitant has the following chemical structure:

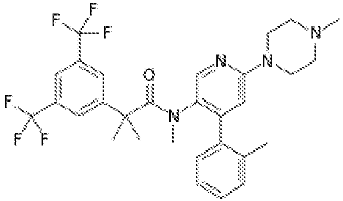

It is used for the prevention of acute and delayed chemotherapy-induced nausea and vomiting, including highly emetogenic chemotherapy such as with cisplatin.

Palonosetron hydrochloride is chemically (3aS)-2-[(S)-1-Azabicyclo [2.2.2]oct-3-yl]- 2,3,3a,4,5,6-hexahydro-1-oxo-1Hbenz[de]isoquinoline hydrochloride. The empirical formula is C₁₉H₂₄N₂O.HCl, with a molecular weight of 332.87. Palonosetron hydrochloride exists as a single isomer and has the following structural formula:

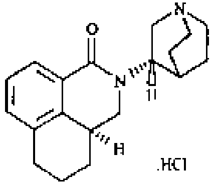

Palonosteron hydrochloride is marketed as Aloxi injection and capsules and is indicated for the treatment of acute and delayed nausea and vomiting associated with initial and repeat courses of moderately emetogenic cancer chemotherapy (MEC); acute nausea and vomiting associated with initial and repeat courses of highly emetogenic cancer chemotherapy (HEC); postoperative nausea and vomiting (PONV) and acute nausea and vomiting associated with initial and repeat courses of emetogenic cancer chemotherapy, including highly emetogenic cancer chemotherapy (HEC).

The formulation of the present invention can be presented in any of the following dosage forms:
(i) liquid (solution or suspension)
(ii) soft gelatin capsule
(iii) liquid filled hard gelatin capsule
(iv) a kit with a liquid concentrate and a diluent
(v) a liquid (solution or suspension) that is diluted with water or any other suitable diluent before administration

### Examples

### A. Nano lipid carrier system compositions

### Example 1: Compositions of nano lipid carrier system NLC (comparative)

| **S. No** | **Ingredients** | **Quantity (%w/v)** |
|---|---|---|
| 1. | decanoic acid | 0.8-1.2 |
| 2. | Labrafac Lipophile WL 1349 (Medium Chain Triglycerides NF) | 0.6-1.2 |
| 3. | N-(Carbonyl methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 0.3-1.2 |
| 4. | ethanol | 0.5-1.0 |
| 5. | egg lecithin (Lipoid E 80S) | 0.5-1.0 |
| 6. | cholesterol | 0.2-0.3 |
| 7. | poloxamer-188 | 0.5-1.0 |
| 8. | vitamin E acetate | 0.01-0.02 |
| 9. | 1,4,7,10-Tetraazacyclododecane-1,4,7,10-tetraacetic acid (DOTA) | 0.001-0.002 |
| 10. | propyl paraben | 0.01-0.02 |
| 11. | chlorbutanol | 0.01-0.025 |
| 12. | tris base | 0.1-0.15 |
| 13. | dextrose anhydrous | 3.5-4.0 |
| 14. | sucralose | 0.05-0.1 |
| 15. | peppermint supreme | 0.05-0.1 |
| 16. | Purified Water | Q.S to 100% |

**Preparation of Oil Phase:** Accurately weighed quantity of vitamin E acetate was taken into a manufacturing vessel. Labrafac Lipophile WL 1349 (oil) was subsequently added to it followed by decanoic acid and dissolved by heating on a water bath at 55°C (50°C to 60°C). Weighed quantities of cholesterol, PEG-PE and egg lecithin (Lipoid E 80S) were further added and dissolved. This mixture was cooled to 40°C, ethanol, propyl paraben and peppermint supreme flavor were added.

**Preparation of Aqueous Phase:** 90% of required quantity of Milli-Q water per batch was taken in another manufacturing vessel in which weighed quantity of poloxamer-188 was added and dissolved by heating at 55°C (50°C to 60°C). dextrose, sucralose and DOTA were subsequently added and dissolved in the above solution.

### Preparation of NLC:

**High Shear Homogenization:** The lipid phase was added into the aqueous phase, under homogenization at 4000 rpm by maintaining the product temperature at 55°C (50°C to 60°C) and the homogenization was continued further for 15 min at 8500 rpm. Later, the volume was adjusted up to required level with remaining quantity of Milli-Q water followed by homogenization at 8500 rpm for 15 minutes for formation of a coarse dispersion.

**High Pressure Homogenization:** The obtained coarse dispersion was subjected to high pressure homogenization for 3 passes at different pressures i.e. Pass 1 at 5,000 psi, Pass 2 at 10,000 psi and Pass 3 at 18,000 psi.

The Size and Zeta Potential of the undiluted NLC dispersion were measured using Particle Size Analyzer (Lite Sizer, Anton Paar).

### Example 2: Oral nanocarrier formulation of netupitant and palonosetron with various PEGylated lipid concentrations (comparative)

| **S. No** | **Batch#** | **135A (0% PEGylated lipid)** | **135C (0.6% PEGylated lipid)** | **135D (1.2% PEGylated lipid)** |
|---|---|---|---|---|
| | **Ingredients** | **% (w/v)** | | |
| 1 | decanoic acid | 1.200 | 1.200 | 1.200 |
| 2 | Labrafac Lipophile WL 1349 (Medium Chain Triglycerides NF) | 1.200 | 1.200 | 1.200 |
| 3 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 0.000 | 0.600 | 1.200 |
| 4 | netupitant | 0.600 | 0.600 | 0.600 |
| 5 | palonosetron hydrochloride | 0.001 | 0.001 | 0.001 |
| 6 | ethanol | 1.000 | 1.000 | 1.000 |
| 7 | soy lecithin (Lipoid S 100) | 0.500 | 0.500 | 0.500 |
| 8 | cholesterol | 0.300 | 0.300 | 0.300 |
| 9 | poloxamer-188 | 1.000 | 1.000 | 1.000 |
| 10 | vitamin E acetate | 0.020 | 0.020 | 0.020 |
| 11 | propyl paraben | 0.020 | 0.020 | 0.020 |
| 12 | 1,4,7,10-Tetraazacyclododecane-1,4,7,10-tetraacetic acid (DOTA) | 0.002 | 0.002 | 0.002 |
| 13 | dextrose anhydrous | 3.500 | 3.500 | 3.500 |
| 14 | sucralose | 0.100 | 0.100 | 0.100 |
| 15 | peppermint supreme | 0.100 | 0.100 | 0.100 |
| 16 | tris base | 0.100 | 0.100 | 0.100 |
| 17 | Milli-Q Water | Q.S to 100% | Q.S to 100% | Q.S to 100% |

### Manufacturing Procedure:

**Preparation of Oil Phase:** Accurately weighed quantity of vitamin E acetate was taken into a manufacturing vessel. Labrafac Lipophile WL 1349 (oil) was subsequently added to it followed by decanoic acid and dissolved by heating on a water bath at 55°C (50°C to 60°C). Weighed quantities of cholesterol, PEG-PE and Soy lecithin (Lipoid S 100) were further added and dissolved. This mixture was cooled to 40°C, ethanol, propyl paraben and peppermint supreme flavor were added. Weighed amount of netupitant was added to the above mixture and dissolved to give a lipid phase.

**Preparation of Aqueous Phase:** 90% of required quantity of Milli-Q water per batch was taken in another manufacturing vessel in which weighed quantity of poloxamer-188 was added and dissolved by heating at 55°C (50°C to 60°C). dextrose, sucralose and DOTA were subsequently added and dissolved in the above solution.

### Preparation of NLC:

**High Shear Homogenization:** The lipid phase was added into the aqueous phase, under homogenization at 4000 rpm by maintaining the product temperature at 55°C (50°C to 60°C) and the homogenization was continued further for 15 min at 8500 rpm. Later, the volume was adjusted up to required level with remaining quantity of Milli-Q water followed by homogenization at 8500 rpm for 15 minutes for formation of a coarse dispersion.

**High Pressure Homogenization:** The obtained coarse dispersion was subjected to high pressure homogenization for 3 passes at different pressures i.e. Pass 1 at 5,000 psi, Pass 2 at 10,000 psi and Pass 3 at 18,000 psi.

Required quantity of palonosetron hydrochloride was added from prepared stock solution after high pressure homogenization. Finally, the pH of the preparation was adjusted in between 6.0 - 6.5 with tris base.

The size and zeta potential of the undiluted NLC dispersion were measured using particle size analyzer (Lite Sizer, Anton Paar).

The nano dispersion obtained was characterised for the description, pH, hydrodynamic diameter and the data is tabulated below:

**Table 2: Characteristics data of the nanodispersion:**

| **Characteristics of the nanodispersion** | **135A** | **135C** | **135D** |
|---|---|---|---|
| Description | Off-white dispersion | Off-white dispersion | Off-white dispersion |
| pH | 6.49 | 6.62 | 6.62 |
| Osmolality (mOsm/kg) | 241 | 227 | 258 |
| Particle size distribution | | | |
| HDD (nm) | 132.24 | 111.18 | 114.28 |
| D10 (nm) | 69.61 | 57.54 | 64.45 |
| D50 (nm) | 128.80 | 115.88 | 116.75 |
| D90 (nm) | 223.65 | 197.43 | 186.70 |
| PDI (%) | 21.37 | 23.17 | 22.0 |
| SPAN | 1.19 | 1.21 | 1.05 |
| Zeta potential (mV) | -11.17 | -8.80 | -10.20 |
| Assay of netupitant (%) | 92.6 | 93.5 | 103.3 |

### Example 3: Oral nanocarrier formulation of netupitant and palonosetron with various PEGylated lipid concentrations and containing cannabidiol.

| **S. No** | **Batch#** | **136A (CBD: 0.4%; PEGylated lipid: 0%)** | **136D (CBD: 0.4%; PEGylated lipid: 0.6%)** | **136E (CBD: 0.4%; PEGylated lipid: 1.2%)** |
|---|---|---|---|---|
| | **Ingredients** | % **(w/v)** | | |
| 1 | decanoic acid | 1.200 | 1.200 | 1.200 |
| 2 | Labrafac Lipophile WL 1349 (Medium Chain Triglycerides NF) | 1.200 | 1.200 | 1.200 |
| 3 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 0.000 | 0.600 | 1.200 |
| 4 | cannabidiol (CBD) | 0.400 | 0.400 | 0.400 |
| 5 | netupitant | 0.600 | 0.600 | 0.600 |
| 6 | palonosetron hydrochloride | 0.001 | 0.001 | 0.001 |
| 7 | ethanol | 1.000 | 1.000 | 1.000 |
| 8 | Egg Lecithin (Lipoid E80S) | 1.000 | 1.000 | 1.000 |
| 9 | cholesterol | 0.300 | 0.300 | 0.300 |
| 10 | poloxamer-188 | 1.000 | 1.000 | 1.000 |
| 11 | vitamin E acetate | 0.020 | 0.020 | 0.020 |
| 12 | ascorbyl palmitate | 0.020 | 0.020 | 0.020 |
| 13 | propyl paraben | 0.020 | 0.020 | 0.020 |
| 14 | 1,4,7,10-Tetraazacyclododecane-1,4,7,10-tetraacetic acid (DOTA) | 0.002 | 0.002 | 0.002 |
| 15 | dextrose anhydrous | 3.500 | 3.500 | 3.500 |
| 16 | sucralose | 0.100 | 0.100 | 0.100 |
| 17 | peppermint supreme | 0.100 | 0.100 | 0.100 |
| 18 | tris base | 0.100 | 0.100 | 0.100 |
| 19 | Milli-Q Water | Q.S to 100% | Q.S to 100% | Q.S to 100% |

### Manufacturing Procedure:

**Preparation of Oil Phase:** Accurately weighed quantity of vitamin E acetate was taken into a manufacturing vessel. Labrafac Lipophile WL 1349 (oil) was subsequently added to it followed by decanoic acid and dissolved by heating on a water bath at 55°C (50°C to 60°C). Weighed quantities of ascorbyl palmitate, cholesterol, PEG-PE and egg lecithin (Lipoid E80S) were further added and dissolved. This mixture was cooled to 40°C, ethanol, propyl paraben and peppermint supreme flavor were added. Weighed amount of netupitant and cannabidiol were added to the above mixture and dissolved to give a lipid phase.

**Preparation of Aqueous Phase:** 90% of required quantity of Milli-Q water per batch was taken in another manufacturing vessel in which weighed quantity of Poloxamer-188 was dissolved by heating at 55°C (50°C to 60°C). Dextrose, sucralose and DOTA were subsequently added and dissolved in the above solution.

### Preparation of NLC:

**High Shear Homogenization:** The lipid phase was added into the aqueous phase, under homogenization at 4000 rpm by maintaining the product temperature at 55°C (50°C to 60°C) and the homogenization was continued further for 15 min at 8500 rpm. Later, the volume was adjusted up to required level with remaining quantity of Milli-Q water followed by homogenization at 8500 rpm for 15 minutes for formation of a coarse dispersion.

**High Pressure Homogenization:** The obtained coarse dispersion was subjected to high pressure homogenization for 3 passes at different pressures i.e. Pass 1 at 5,000 psi, Pass 2 at 10,000 psi and Pass 3 at 18,000 psi.

Required quantity of palonosetron hydrochloride was added from prepared stock solution after high pressure homogenization. Finally, the pH of the preparation was adjusted in between 6.0 - 6.5 with tris base. The size and zeta potential of the undiluted NLC dispersion were measured using particle size analyzer (Lite Sizer, Anton Paar).

**Table 3: Characteristics data of the nanodispersion**

| **Characteristics of the nanodispersion** | | | |
|---|---|---|---|
| Description | Off-white dispersion | Off-white dispersion | Off-white dispersion |
| pH | 6.57 | 6.68 | 6.70 |
| Osmolality (mOsm/kg) | 220 | 213 | 232 |
| Particle Size Distribution | | | |
| HDD (nm) | 214.01 | 135.62 | 158.03 |
| D10 (nm) | 134.18 | 67.07 | 79.96 |
| D50 (nm) | 196.82 | 136.58 | 166.52 |
| D90 (nm) | 284.31 | 244.0 | 277.88 |
| PDI (%) | 12.90 | 22.53 | 25.93 |
| SPAN | 0.76 | 1.29 | 1.19 |
| Zeta Potential (mV) | -28.50 | -13.87 | -9.73 |
| Assay of Netupitant (%) | 93.7 | 90.3 | 99.4 |
| Assay of CBD (%) | 90.1 | 93.8 | 97.0 |

As the concentration of PEG-PE increased in the NLC formulations, decrease in the Zeta Potential was noted which indicates effective shielding of NLC with PEG-PE. The Clarity of the NLC formulations increased with increase in the concentration of PEG-PE.

### Example 4: Preparation of cannabidiol-IH formulation for comparative evaluation of permeability:

The example 1 of published patent # WO 2015/193668 A1 was reproduced in the laboratory. The composition reproduced from the patent for evaluating comparative permeability study is described below:

### Composition of CBD-IH Oral preparation

| **Ingredients** | **CBD Patent*** | **CBD-IH** |
|---|---|---|
| cannabidiol (CBD) | 100 mg/mL | 100 mg/mL |
| anhydrous ethanol | 79.0 mg/mL | 79.0 mg/mL |
| sucralose | 0.5 mg/mL | 0.5 mg/mL |
| strawberry flavor | 0.2 mg/mL | - |
| sesame oil | q.s to 1.0 mL | sesame oil q.s to 1.0 mL |

The composition disclosed in WO 2015/193668 Al (Example 1) was reproduced in the lab to be used as a control sample.

### Method of Preparation of CBD-IH:

Weighed quantity of anhydrous ethanol was taken into a vial, to which cannabidiol and sucralose was added and dissolved. Required quantity of sesame oil was added to the alcoholic mixture and vortexed for 2 min to give a homogenous product.

### PERMEATION STUDY BY IN VITRO NON-EVERTED RAT INTESTINAL SAC MODEL

Permeation study was conducted by non-everted rat intestinal sac model. The non-everted gut sac method involves placing the test sample in the rat gut sac without everting. The non-everted gut sac was prepared by removing the small intestine section from overnight fasted anaesthetised rats. The intestine was excised and rinsed with ringer lactate solution at room temperature under aeration. The jejunum was knotted at one end, filled with aliquot volume of test sample and the other end was knotted. This intestine bag or sac (considered as donor compartment) was placed in a buffer solution (receptor compartment) maintained at 37°C and at 100 rpm.
(1) Volume of test sample taken into the Intestinal sac i.e., donor compartment - 2 mL
(2) Volume of the buffer medium taken in receptor compartment - 75 mL
(3) Buffer solution (receptor compartment) - pH 6.8 Phosphate buffer (With 1% (w/v) Solutol HS15)
(4) Time Points at which samples were collected - 0.25h, 0.5h, 1 h, 2h, 3h, 4h, 6h, 9h, 12h and 24h.
(5) Volume of the sample collected at each time point - 10 mL

**Table 4: Test samples used in the permeation study**

| **S. No** | **Batch #** | **Description** |
|---|---|---|
| 1 | B#136G | Akynzeo* (Used as control) |
| 2 | B#119C | CBD Oral Preparation (CBD-IH)** (Used as control) |
| 3 | B#135A | NEPA NLC with 0 % PEGylated lipid |
| 4 | B#135C | NEPA NLC with 0.6 % PEGylated lipid |
| 5 | B#135D | NEPA NLC with 1.2 % PEGylated lipid |
| 6 | B#136A | NEPA+ cannabidiol NLC with 0 % PEGylated lipid |
| 7 | B#136D | NEPA+ cannabidiol NLC with 0.6 % PEGylated lipid |
| 8 | B#136E | NEPA+ cannabidiol NLC with 1.2 % PEGylated lipid |
| 9 | B#136C | NEPA self-dispersible NLC concentrate*** with 4.6 % PEGylated lipid |

| | | |
|---|---|---|
| Note: *Akynzeo capsule (Batch No: 39002435; Expiry Date: 02/2022) was completely dispersed in 50 mL volumetric flask with pH 6.8 Phosphate buffer by vortex mixing. **CBD Oral Preparation (CBD-IH) (40 µL was taken and was dispersed in pH 6.8 Phosphate buffer, volume made up to 2mL) *** (130 mg of SDNLC (self-dispersible nano lipid carrier system) was taken and dispersed in pH 6.8 Phosphate buffer, volume made up to 2mL) | | |

The samples were withdrawn from the acceptor compartment at the predetermined time intervals. The samples were analyzed by HPLC and cumulative amount of active ingredient released from unit surface area of intestine was calculated. A graph was plotted between cumulative amount of active ingredient released from unit surface area of intestine and time. The slope of the graph gives the permeation flux rate in (mcg/cm²/h).

The flux rate was calculated for netupitant from NEPA NLC and for both netupitant and CBD from NEPA+CBD NLC which are tabulated in Table 5 to Table 8.

**Method for Determination of Entrapment Efficiency:** 2 mL of NLC preparation was taken into 100 KD MWCO Filter and centrifuged at 4500 rpm for 1h. The upper lipid layer obtained was suitably diluted in assay diluent and the amount of active ingredient entrapped in the lipid was determined by HPLC.

**Table 5: Comparative permeability flux of netupitant from formulations**

| **S. No** | **Formulation** | **netupitant flux rate (mcg/cm²/h)** | **R² value from the graph** | **EE (%)** | **HDD (nm)** | **ZP (mV)** |
|---|---|---|---|---|---|---|
| 1 | Akynzeo (B#136G) | 1.208 | 0.99 | NA | 1396.17 | -10.13 |
| 2 | NEPA NLC with 0 % PEGylated lipid (B#135A) | 1.170 | 0.85 | 93.4 | 132.24 | -11.17 |
| 3 | NEPA NLC with 0.6 % PEGylated lipid (B#135C) | 2.296 | 0.97 | 88.8 | 111.18 | -8.80 |
| 4 | NEPA NLC with 1.2 % PEGylated lipid (B#135D) | 4.555 | 0.93 | 84.3 | 114.28 | -10.20 |

| | | | | | | |
|---|---|---|---|---|---|---|
| EE:entrapment efficiency; Zp: zetapotential; HDD: hydrodynamic diameter | | | | | | |

**Table 6: Comparative permeability flux of netupitant and CBD from formulations**

| **S.No** | **Formulation** | **netupitant** | | **cannabidiol (CBD)** | | **EE (%)** | | | |
|---|---|---|---|---|---|---|---|---|---|
| | | Flux rate (mcg /cm2 /h) | R2 value from the grap h | Flux rate (mcg /cm2 /h) | R2 value from the graph | netup itant | cannab idiol | **HDD (nm)** | **Zp (mV)** |
| 1 | Akynzeo (B#136G) | 1.2 | 0.98 | NA | NA | NA | NA | 1396 .1 | -10.1 |
| 2 | CBD Oral Preparation (CBD-IH) (B# 119C) | NA | NA | 0.15 | 0.86 | NA | NA | NA | NA |
| 3 | NEPA+CBD NLC with 0 % PEGylated lipid (B# 136A) | 1.2 | 0.97 | 0.63 | 0.96 | 94.1 | 87.4 | 214. 0 | -28.5 |
| 4 | NEPA+CBD NLC with 0.6% PEGylated lipid (B#136D) | 4.5 | 0.85 | 3.39 | 0.87 | 89.5 | 85.6 | 135. 6 | -13.8 |
| 5 | NEPA+CBD NLC with 1.2 % PEGylated lipid (B#136E) | 7.9 | 0.98 | 5.70 | 0.99 | 98.2 | 93.1 | 158. 0 | -9.73 |

| | | | | | | | | | |
|---|---|---|---|---|---|---|---|---|---|
| EE:entrapment efficiency; Zp: zetapotential; HDD: hydrodynamic diameter | | | | | | | | | |

**Table 7: Summary of comparative permeability of netupitant from different NLC formulations**

| **S. No** | **Formulation** | **Netupitant** | | | | |
|---|---|---|---|---|---|---|
| | | **Flux rate (mcg/cm²/h)** | **R2 value from the graph** | **netupitant EE (%)** | **HDD (nm)** | **Zp (mV)** |
| 1 | Akynzeo (B#136G) | 1.208 | 0.999 | NA | 1396.17 | -10.13 |
| 2 | NEPA NLC with 0 % PEGylated lipid (B#135A) | 1.170 | 0.855 | 93.4 | 132.24 | -11.17 |
| 3 | NEPA+CBD NLC with 0 % PEGylated lipid (B#136A) | 1.288 | 0.979 | 94.1 | 214.01 | -28.50 |
| 4 | NEPA NLC with 0.6 % PEGylated lipid (B#135C) | 2.296 | 0.979 | 88.8 | 111.18 | -8.80 |
| 5 | NEPA+CBD NLC with 0.6% PEGylated lipid (B#136D) | 4.560 | 0.850 | 89.5 | 135.62 | -13.87 |
| 6 | NEPA NLC with 1.2 % PEGylated lipid (B#135D) | 4.555 | 0.930 | 84.3 | 114.28 | -10.20 |
| 7 | NEPA+CBD NLC with 1.2 % PEGylated lipid (B#136E) | 7.909 | 0.982 | 98.2 | 158.03 | -9.73 |

| | | | | | | |
|---|---|---|---|---|---|---|
| EE:entrapment efficiency; Zp: zetapotential; HDD: hydrodynamic diameter | | | | | | |

**Table 8: Data representing the synergistic effect of PEG-PE and CBD on the permeation of netupitant**

| **S. No** | **Formula tion details** | **PEGyl ated lipid (%w/v)** | **CBD (%w/ v)** | **NEPA flux rate (mcg/cm ²/h)** | **CBD flux rate (mcg/cm ²/h)** | **netupitant flux change** | **CBD flux chang e** |
|---|---|---|---|---|---|---|---|
| 1 | Akynzeo (control) | Nil | Nil | 1.208 | Nil | - | - |
| 2 | B#119C CBD-IH (control) | Nil | 10% | Nil | 0.153 | - | - |
| 3 | B#135A NEPA NLC without PEGylat ed lipid | 0% | Nil | 1.170 | Nil | Comparable to Akynzeo | - |
| 4 | B#135C NEPA NLC with PEGylat ed lipid | 0.6% | Nil | 2.296 | Nil | 1.9 times > than Akynzeo | - |
| 5 | B#135D NEPA NLC with PEGylat ed lipid | 1.2% | Nil | 4.555 | Nil | 3.77 times > than Akynzeo | - |
| 6 | B#136A NEPA NLC without PEGylat ed lipid | 0% | 0.4% | 1.288 | 0.638 | Comparable to Akynzeo | 4.1 times > than CBD-IH |
| 7 | B#136D NEPA NLC with PEGylat ed lipid | 0.6% | 0.4% | 4.560 | 3.390 | 3.7 times > than Akynzeo | 22.1 times > than CBD-IH |
| 8 | B#136E NEPA NLC with PEGylat ed Lipid | 1.2% | 0.4% | 7.909 | 5.706 | 6.5 times > than Akynzeo | 37.3 times > than CBD-IH |

### B. Self-dispersible nanocarrier concentrate compositions in capsules

### Example 5: CBD oral self-dispersible NLC concentrate

| **S. No** | **Ingredients** | **Quantity (mg)** | **%w/v** |
|---|---|---|---|
| 1 | cannabidiol synthetic | 150 | 28.04 |
| 2 | decanoic acid | 75 | 14.02 |
| 3 | egg lecithin | 50 | 9.35 |
| 4 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 30 | 5.61 |
| 5 | Labrafac Lipophile WL 1349 (Medium Chain Triglycerides NF) | 100 | 18.69 |
| 6 | Kolliphor RH40 | 95 | 17.76 |

### Manufacturing Procedure:

Accurately weighed quantity of Labrafac Lipophile WL 1349 (Medium Chain Triglycerides NF) and Kolliphor RH 40 were taken into a glass vial to which decanoic acid, PEG-PE and egg lecithin were added and the contents were dissolved by heating on a water bath at 40°C. Then, required quantity of cannabidiol was added to the above mixture and dissolved by further heating at 40°C to give a homogenous matrix.

The obtained mixture can be encapsulated in hard or soft gelatin capsules.

### Example 6: CBD oral self-dispersible NLC concentrate

| **S. No** | **Ingredients** | **Quantity (mg)** | **%w/v** |
|---|---|---|---|
| 1 | cannabidiol synthetic | 150 | 28.04 |
| 2 | decanoic acid | 75 | 14.02 |
| 3 | egg lecithin | 50 | 9.35 |
| 4 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 30 | 5.61 |
| 5 | caprylic acid | 100 | 18.69 |
| 6 | Kolliphor RH40 | 95 | 17.76 |

### Manufacturing Procedure:

Accurately weighed quantity of caprylic acid and Kolliphor RH 40 were taken into a glass vial to which decanoic acid, PEG-PE and egg lecithin were added and the contents were dissolved by heating on a water bath at 40°C. Then, required quantity of cannabidiol was added to the above mixture and dissolved by further heating at 40°C to give a homogenous matrix.

The obtained mixture can be encapsulated in hard or soft gelatin capsules.

### Example 7: CBD oral self-dispersible NLC concentrate

| **S. No** | **Ingredients** | **Quantity (mg)** | **%w/v** |
|---|---|---|---|
| 1 | cannabidiol synthetic | 150 | 28.04 |
| 2 | decanoic acid | 75 | 14.02 |
| 3 | egg lecithin | 50 | 9.35 |
| 4 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 30 | 5.61 |
| 5 | Incromega E3322 (Fish Oil) | 100 | 18.69 |
| 6 | Kolliphor RH40 | 95 | 17.76 |

### Manufacturing Procedure:

Accurately weighed quantity of Incromega E3322 and Kolliphor RH 40 were taken into a glass vial to which decanoic acid, PEG-PE and egg lecithin were added and the contents were dissolved by heating on a water bath at 40°C. Then, required quantity of cannabidiol was added to the above mixture and dissolved by further heating at 40°C to give a homogenous matrix.

The obtained mixture can be encapsulated in hard or soft gelatin capsules.

### Example 8: Example of CBD oral self-dispersible NLC concentrate

| **S. No** | **Ingredients** | **Quantity (mg)** | **%w/v** |
|---|---|---|---|
| 1 | cannabidiol synthetic | 150 | 28.04 |
| 2 | decanoic acid | 75 | 14.02 |
| 3 | egg lecithin | 50 | 9.35 |
| 4 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 30 | 5.61 |
| 5 | eicosapentaenoic acid (EPA) | 100 | 18.69 |
| 6 | Kolliphor RH40 | 95 | 17.76 |

### Manufacturing Procedure:

Accurately weighed quantity of eicosapentaenoic acid (EPA) and Kolliphor RH 40 were taken into a glass vial to which decanoic acid, PEG-PE and egg lecithin were added and the contents were dissolved by heating on a water bath at 40°C. Then, required quantity of cannabidiol was added to the above mixture and dissolved by further heating at 40°C to give a homogenous matrix.

The obtained mixture can be encapsulated in hard or soft gelatin capsules.

### Example 9: CBD oral self-dispersible NLC concentrate

| **S. No** | **Ingredients** | **Quantity (mg)** | **(%w/v)** |
|---|---|---|---|
| 1 | cannabidiol synthetic | 150 | 28.04 |
| 2 | decanoic acid | 75 | 14.02 |
| 3 | egg lecithin | 50 | 9.35 |
| 4 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 30 | 5.61 |
| 5 | docosahexaenoic acid (DHA) | 100 | 18.69 |
| 6 | Kolliphor RH40 | 95 | 17.76 |

### Manufacturing Procedure:

Accurately weighed quantity of docosahexaenoic acid (DHA) and Kolliphor RH 40 were taken into a glass vial to which decanoic acid, PEG-PE and egg lecithin were added and the contents were dissolved by heating on a water bath at 40°C. Then, required quantity of cannabidiol was added to the above mixture and dissolved by further heating at 40°C to give a homogenous matrix.

The obtained mixture can be encapsulated in hard or soft gelatin capsules.

### C. Orally administered dispersible liquid oil compositions

### Example 10: Self-dispersible cannabidiol oral liquid oil

| **S. No** | **Ingredients** | **Quantity (mg)** | **%w/v** |
|---|---|---|---|
| 1 | cannabidiol synthetic | 150 | 14.02 |
| 2 | grapeseed oil | 300 | 28.04 |
| 3 | decanoic acid | 50 | 4.67 |
| 4 | egg lecithin | 50 | 4.67 |
| 5 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 0.3 | 0.03 |
| 6 | neotame | 30 | 2.80 |
| 7 | peppermint supreme | 20 | 1.87 |
| 8 | spearmint oil | 20 | 1.87 |
| 9 | cinnamon oil | 15 | 1.40 |
| 10 | menthol | 10 | 0.93 |
| 11 | ethanol | 100 | 9.35 |
| 12 | Kolliphor RH40 | 100 | 9.35 |
| 13 | Labrafac Lipophile WL 1349 (Medium Chain Triglycerides NF) | 155 | 14.49 |

### Manufacturing Procedure:

Accurately weighed quantity of Labrafac Lipophile WL 1349 (Medium Chain Triglycerides NF), grapeseed oil and Kolliphor RH40 were taken into a glass vial to which decanoic acid, PEG-PE and Egg lecithin were added and the contents were dissolved by heating on a water bath at 40°C. Then, required quantity of cannabidiol was added to the above mixture and dissolved by further heating at 40°C to give a homogenous oil matrix which was then cooled to room temperature.

In another vial weighed quantity of ethanol was taken to which neotame, menthol, spearmint oil, cinnamon oil and peppermint supreme were added and dissolved by stirring for 5 min at 400 rpm. The ethanolic mixture of this vial was transferred to the vial containing cooled oil matrix and the resultant mixture was mixed for 2 min at 400 rpm.

### Example 11: Self-dispersible cannabidiol oral liquid oil

| **S. No** | **Ingredients** | **Quantity (mg)** | **%w/v** |
|---|---|---|---|
| 1 | cannabidiol synthetic | 150 | 14.02 |
| 2 | grapeseed oil | 300 | 28.04 |
| 3 | decanoic acid | 50 | 4.67 |
| 4 | egg lecithin | 50 | 4.67 |
| 5 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 30 | 2.80 |
| 6 | neotame | 30 | 2.80 |
| 7 | peppermint supreme | 20 | 1.87 |
| 8 | spearmint oil | 20 | 1.87 |
| 9 | cinnamon oil | 15 | 1.40 |
| 10 | menthol | 10 | 0.93 |
| 11 | ethanol | 100 | 9.35 |
| 12 | Kolliphor RH40 | 100 | 9.35 |
| 13 | Labrafac Lipophile WL 1349 (Medium Chain Triglycerides NF) | 125 | 11.68 |

### Manufacturing Procedure:

Accurately weighed quantity of Labrafac Lipophile WL 1349 (Medium Chain Triglycerides NF), grapeseed oil and Kolliphor RH40 were taken into a glass vial to which decanoic acid, PEG-PE and egg lecithin were added and the contents were dissolved by heating on a water bath at 40°C. Then, required quantity of cannabidiol was added to the above mixture and dissolved by further heating at 40°C to give a homogenous oil matrix which was then cooled to room temperature.

In another vial weighed quantity of ethanol was taken to which neotame, menthol, spearmint oil, cinnamon oil and peppermint supreme were added and dissolved by stirring for 5 min at 400 rpm. The ethanolic mixture of this vial was transferred to the vial containing cooled oil matrix and the resultant mixture was mixed for 2 min at 400 rpm.

### Example 12: Self-dispersible cannabidiol oral liquid oil

| **S. No** | **Ingredients** | **Quantity (mg)** | **%w/v** |
|---|---|---|---|
| 1 | cannabidiol synthetic | 150 | 14.02 |
| 2 | grapeseed oil | 300 | 28.04 |
| 3 | decanoic acid | 50 | 4.67 |
| 4 | egg lecithin | 50 | 4.67 |
| 5 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 100 | 9.35 |
| 6 | neotame | 30 | 2.80 |
| 7 | peppermint supreme | 20 | 1.87 |
| 8 | spearmint oil | 20 | 1.87 |
| 9 | cinnamon oil | 15 | 1.40 |
| 10 | menthol | 10 | 0.93 |
| 11 | ethanol | 100 | 9.35 |
| 12 | Kolliphor RH40 | 100 | 9.35 |
| 13 | Labrafac Lipophile WL 1349 (Medium Chain Triglycerides NF) | 55 | 5.14 |

### Manufacturing Procedure:

Accurately weighed quantity of Labrafac Lipophile WL 1349 (Medium Chain Triglycerides NF), grapeseed oil and Kolliphor RH40 were taken into a glass vial to which decanoic acid, PEG-PE and egg lecithin were added and the contents were dissolved by heating on a water bath at 40°C. Then, required quantity of cannabidiol was added to the above mixture and dissolved by further heating at 40°C to give a homogenous oil matrix which was then cooled to room temperature.

In another vial weighed quantity of ethanol was taken to which neotame, menthol, spearmint oil, cinnamon oil and peppermint supreme were added and dissolved by stirring for 5 min at 400 rpm. The ethanolic mixture of this vial was transferred to the vial containing cooled oil matrix and the resultant mixture was mixed for 2 min at 400 rpm.

### Example 13: Self-dispersible cannabidiol oral liquid oil

| **S. No** | **Ingredients** | **Quantity (mg)** | **%w/v** |
|---|---|---|---|
| 1 | cannabidiol synthetic | 150 | 14.02 |
| 2 | grapeseed oil | 300 | 28.04 |
| 3 | decanoic acid | 50 | 4.67 |
| 4 | egg lecithin | 50 | 4.67 |
| 5 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 0.3 | 0.03 |
| 6 | neotame | 30 | 2.80 |
| 7 | peppermint supreme | 20 | 1.87 |
| 8 | spearmint oil | 20 | 1.87 |
| 9 | cinnamon oil | 15 | 1.40 |
| 10 | menthol | 10 | 0.93 |
| 11 | ethanol | 100 | 9.35 |
| 12 | sucrose laurate | 100 | 9.35 |
| 13 | Labrafac Lipophile WL 1349 (Medium Chain Triglycerides NF) | 155 | 14.49 |

### Manufacturing Procedure:

Accurately weighed quantity of Labrafac Lipophile WL 1349 (Medium Chain Triglycerides NF) and grapeseed oil were taken into a glass vial to which decanoic acid, PEG-PE and egg lecithin were added and the contents were dissolved by heating on a water bath at 40°C. Then, required quantity of cannabidiol was added to the above mixture and dissolved by further heating at 40°C to give a homogenous oil matrix which was then cooled to room temperature.

In another vial weighed quantity of ethanol was taken to which sucrose laurate, neotame, menthol, spearmint oil, cinnamon oil and peppermint supreme were added and dissolved by stirring for 5 min at 400 rpm. The ethanolic mixture of this vial was transferred to the vial containing cooled oil matrix and the resultant mixture was mixed for 2 min at 400 rpm.

### Example 14: Self -dispersible cannabidiol oral liquid oil

| **S. No** | **Ingredients** | **Quantity (mg)** | **%w/v** |
|---|---|---|---|
| 1 | cannabidiol synthetic | 150 | 14.02 |
| 2 | grapeseed oil | 300 | 28.04 |
| 3 | decanoic acid | 50 | 4.67 |
| 4 | egg lecithin | 50 | 4.67 |
| 5 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 30 | 2.80 |
| 6 | neotame | 30 | 2.80 |
| 7 | peppermint supreme | 20 | 1.87 |
| 8 | spearmint oil | 20 | 1.87 |
| 9 | cinnamon oil | 15 | 1.40 |
| 10 | menthol | 10 | 0.93 |
| 11 | ethanol | 100 | 9.35 |
| 12 | sucrose laurate | 100 | 9.35 |
| 13 | Labrafac Lipophile WL 1349 (Medium Chain Triglycerides NF) | 125 | 11.68 |

### Manufacturing Procedure:

Accurately weighed quantity of Labrafac Lipophile WL 1349 (Medium Chain Triglycerides NF) and grapeseed oil were taken into a glass vial to which decanoic acid, PEG-PE and egg lecithin were added and the contents were dissolved by heating on a water bath at 40°C. Then, required quantity of cannabidiol was added to the above mixture and dissolved by further heating at 40°C to give a homogenous oil matrix which was then cooled to room temperature.

In another vial weighed quantity of ethanol was taken to which sucrose laurate, neotame, menthol, spearmint oil, cinnamon oil and peppermint supreme were added and dissolved by stirring for 5 min at 400 rpm. The ethanolic mixture of this vial was transferred to the vial containing cooled oil matrix and the resultant mixture was mixed for 2 min at 400 rpm.

### Example 15: Self- dispersible cannabidiol oral liquid oil

| **S. No** | **Ingredients** | **Quantity (mg)** | **%w/v** |
|---|---|---|---|
| 1 | cannabidiol synthetic | 150 | 14.02 |
| 2 | grapeseed oil | 300 | 28.04 |
| 3 | decanoic acid | 50 | 4.67 |
| 4 | egg lecithin | 50 | 4.67 |
| 5 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 100 | 9.35 |
| 6 | neotame | 30 | 2.80 |
| 7 | peppermint supreme | 20 | 1.87 |
| 8 | spearmint oil | 20 | 1.87 |
| 9 | cinnamon oil | 15 | 1.40 |
| 10 | menthol | 10 | 0.93 |
| 11 | ethanol | 100 | 9.35 |
| 12 | sucrose laurate | 100 | 9.35 |
| 13 | Labrafac Lipophile WL 1349 (Medium Chain Triglycerides NF) | 55 | 5.14 |

### Manufacturing Procedure:

Accurately weighed quantity of Labrafac Lipophile WL 1349 (Medium Chain Triglycerides NF) and grapeseed oil were taken into a glass vial to which decanoic acid, PEG-PE and egg lecithin were added and the contents were dissolved by heating on a water bath at 40°C. Then, required quantity of cannabidiol was added to the above mixture and dissolved by further heating at 40°C to give a homogenous oil matrix which was then cooled to room temperature.

In another vial weighed quantity of ethanol was taken to which sucrose laurate, neotame, menthol, spearmint oil, cinnamon oil and peppermint supreme were added and dissolved by stirring for 5 min at 400 rpm. The ethanolic mixture of this vial was transferred to the vial containing cooled oil matrix and the resultant mixture was mixed for 2 min at 400 rpm.

### Example 16: Self -dispersible netupitant and palonosetron oral liquid oil (comparative)

| **S. No** | **Ingredients** | **Quantity (mg)** | **%w/v** |
|---|---|---|---|
| 1 | netupitant | 100 | 9.35 |
| 2 | palonosetron hydrochloride | 0.167 | 0.02 |
| 3 | grapeseed oil | 300 | 28.04 |
| 4 | decanoic acid | 50 | 4.67 |
| 5 | egg lecithin | 50 | 4.67 |
| 6 | N-(Carbonyl- ethoxypolyethylenglycol-2000)-1,2-distearoyl-sn-glycero-3-phosphoethanolamine, MPEG-2000-DSPE, Na-salt (Lipoid PE 18:0/18:0-PEG 2000) | 30 | 2.80 |
| 7 | neotame | 30 | 2.80 |
| 8 | peppermint supreme | 20 | 1.87 |
| 9 | spearmint oil | 20 | 1.87 |
| 10 | cinnamon oil | 15 | 1.40 |
| 11 | menthol | 10 | 0.93 |
| 12 | ethanol | 100 | 9.35 |
| 13 | propylene glycol | 25 | 2.34 |
| 14 | Tween-80 | 100 | 9.35 |
| 15 | Labrafac Lipophile WL 1349 (Medium Chain Triglycerides NF) | 150 | 14.02 |

### Manufacturing Procedure:

Accurately weighed quantity of Labrafac Lipophile WL 1349 (Medium Chain Triglycerides NF), Tween-80 and grapeseed oil were taken into a glass vial to which decanoic acid, PEG-PE and egg lecithin were added and the contents were dissolved by heating on a water bath at 40°C. Then, required quantity of netupitant was added to the above mixture and dissolved by further heating at 40°C to give a homogenous oil matrix which was then cooled to room temperature.

In another vial weighed quantity of propylene glycol was taken and palonosetron hydrochloride was dissolved in it followed by ethanol to which neotame, menthol, spearmint oil, cinnamon oil and peppermint supreme were added and dissolved by stirring for 5 min at 400 rpm. The ethanolic mixture of this vial was transferred to the vial containing cooled oil matrix and the resultant mixture was mixed for 2 min at 400 rpm.

### Example 17: Self-dispersible netupitant, cannabidiol and palonosetron oral liquid oil

| **S. No** | **Ingredients** | **Quantity (mg)** | **%w/v** |
|---|---|---|---|
| 1 | netupitant | 100 | 9.35 |
| 2 | palonosetron hydrochloride | 0.167 | 0.02 |
| 3 | cannabidiol | 50 | 4.67 |
| 4 | grapeseed oil | 300 | 28.04 |
| 5 | decanoic acid | 50 | 4.67 |
| 6 | egg lecithin | 50 | 4.67 |
| 7 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 0.3 | 0.03 |
| 8 | neotame | 30 | 2.80 |
| 9 | peppermint supreme | 20 | 1.87 |
| 10 | spearmint oil | 20 | 1.87 |
| 11 | cinnamon oil | 15 | 1.40 |
| 12 | menthol | 10 | 0.93 |
| 13 | ethanol | 100 | 9.35 |
| 14 | propylene glycol | 25 | 2.34 |
| 15 | Tween-80 | 100 | 9.35 |
| 16 | Labrafac Lipophile WL 1349 (Medium Chain Triglycerides NF) | 130 | 12.15 |

### Manufacturing Procedure:

Accurately weighed quantity of Labrafac Lipophile WL 1349 (Medium Chain Triglycerides NF), Tween-80 and grapeseed oil were taken into a glass vial to which decanoic acid, PEG-PE and egg lecithin were added and the contents were dissolved by heating on a water bath at 40°C. Then, required quantity of cannabidiol followed by netupitant were added to the above mixture and dissolved by further heating at 40°C to give a homogenous oil matrix which was then cooled to room temperature.

In another vial weighed quantity of propylene glycol was taken and palonosetron hydrochloride was dissolved in it followed by ethanol to which neotame, menthol, spearmint oil, cinnamon oil and peppermint supreme were added and dissolved by stirring for 5 min at 400 rpm. The ethanolic mixture of this vial was transferred to the vial containing cooled oil matrix and the resultant mixture was mixed for 2 min at 400 rpm.

### Example 18: Self-dispersible netupitant, cannabidiol and palonosetron oral liquid oil

| **S. No** | **Ingredients** | **Quantity (mg)** | **%w/v** |
|---|---|---|---|
| 1 | netupitant | 100 | 9.35 |
| 2 | palonosetron hydrochloride | 0.167 | 0.02 |
| 3 | cannabidiol | 50 | 4.67 |
| 4 | grapeseed oil | 300 | 28.04 |
| 5 | decanoic acid | 50 | 4.67 |
| 6 | egg lecithin | 50 | 4.67 |
| 7 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 30 | 2.80 |
| 8 | neotame | 30 | 2.80 |
| 9 | peppermint supreme | 20 | 1.87 |
| 10 | spearmint oil | 20 | 1.87 |
| 11 | cinnamon oil | 15 | 1.40 |
| 12 | menthol | 10 | 0.93 |
| 13 | ethanol | 100 | 9.35 |
| 14 | propylene glycol | 25 | 2.34 |
| 15 | Tween-80 | 100 | 9.35 |
| 16 | Labrafac Lipophile WL 1349 (Medium Chain Triglycerides NF) | 100 | 9.35 |

### Manufacturing Procedure:

Accurately weighed quantity of Labrafac Lipophile WL 1349 (Medium Chain Triglycerides NF), Tween-80 and grapeseed oil were taken into a glass vial to which decanoic acid, PEG-PE and egg lecithin were added and the contents were dissolved by heating on a water bath at 40°C. Then, required quantity of cannabidiol followed by netupitant were added to the above mixture and dissolved by further heating at 40°C to give a homogenous oil matrix which was then cooled to room temperature.

In another vial weighed quantity of propylene glycol was taken and palonosetron hydrochloride was dissolved in it followed by ethanol to which neotame, menthol, spearmint oil, cinnamon oil and peppermint supreme were added and dissolved by stirring for 5 min at 400 rpm. The ethanolic mixture of this vial was transferred to the vial containing cooled oil matrix and the resultant mixture was mixed for 2 min at 400 rpm.

### Example 19: Self -dispersible netupitant, cannabidiol and palonosetron oral liquid oil

| **S. No** | **Ingredients** | **Quantity (mg)** | **%w/v** |
|---|---|---|---|
| 1 | netupitant | 100 | 9.35 |
| 2 | palonosetron hydrochloride | 0.167 | 0.02 |
| 3 | cannabidiol | 50 | 4.67 |
| 4 | grapeseed oil | 300 | 28.04 |
| 5 | decanoic acid | 50 | 4.67 |
| 6 | egg lecithin | 50 | 4.67 |
| 7 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 100 | 9.35 |
| 8 | neotame | 30 | 2.80 |
| 9 | peppermint supreme | 20 | 1.87 |
| 10 | spearmint oil | 20 | 1.87 |
| 11 | cinnamon oil | 15 | 1.40 |
| 12 | menthol | 10 | 0.93 |
| 13 | ethanol | 100 | 9.35 |
| 14 | propylene glycol | 25 | 2.34 |
| 15 | Tween-80 | 100 | 9.35 |
| 16 | Labrafac Lipophile WL 1349 (Medium Chain Triglycerides NF) | 30 | 2.80 |

### Manufacturing Procedure:

Accurately weighed quantity of Labrafac Lipophile WL 1349 (Medium Chain Triglycerides NF), Tween-80 and grapeseed oil were taken into a glass vial to which decanoic acid, PEG-PE and egg lecithin were added and the contents were dissolved by heating on a water bath at 40°C. Then, required quantity of cannabidiol followed by netupitant were added to the above mixture and dissolved by further heating at 40°C to give a homogenous oil matrix which was then cooled to room temperature.

In another vial weighed quantity of propylene glycol was taken and palonosetron hydrochloride was dissolved in it followed by ethanol to which neotame, menthol, spearmint oil, cinnamon oil and peppermint supreme were added and dissolved by stirring for 5 min at 400 rpm. The ethanolic mixture of this vial was transferred to the vial containing cooled oil matrix and the resultant mixture was mixed for 2 min at 400 rpm.

### Example 20: Self dispersible gabapentin oral liquid oil (comparative)

| **S. No** | **Ingredients** | **Quantity (mg)** | **%w/v** |
|---|---|---|---|
| 1 | gabapentin | 100 | 9.35 |
| 2 | omega fatty acid | 475 | 44.39 |
| 3 | decanoic acid | 50 | 4.67 |
| 4 | egg lecithin | 50 | 4.67 |
| 5 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 30 | 2.80 |
| 6 | neotame | 30 | 2.80 |
| 7 | peppermint supreme | 20 | 1.87 |
| 8 | spearmint oil | 20 | 1.87 |
| 9 | cinnamon oil | 15 | 1.40 |
| 10 | menthol | 10 | 0.93 |
| 11 | ethanol | 100 | 9.35 |
| 12 | Tween-80 | 100 | 9.35 |

### Manufacturing Procedure:

Accurately weighed quantity of omega fatty acid and Tween-80 were taken into a glass vial to which decanoic acid, PEG-PE and egg lecithin were added and the contents were dissolved by heating on a water bath at 40°C. Then, required quantity of gabapentin was added to the above mixture and dissolved by further heating at 40°C to give a homogenous oil matrix which was then cooled to room temperature.

In another vial weighed quantity of ethanol was taken to which neotame, menthol, spearmint oil, cinnamon oil and peppermint supreme were added and dissolved by stirring for 5 min at 400 rpm. The ethanolic mixture of this vial was transferred to the vial containing cooled oil matrix and the resultant mixture was mixed for 2 min at 400 rpm.

### Example 21: Self dispersible gabapentin oral liquid oil

| **S. No** | **Ingredients** | **Quantity (mg)** | **%w/v** |
|---|---|---|---|
| 1 | gabapentin | 100 | 9.35 |
| 2 | cannabidiol | 50 | 4.67 |
| 3 | omega fatty acid | 425 | 39.72 |
| 4 | decanoic acid | 50 | 4.67 |
| 5 | egg lecithin | 50 | 4.67 |
| 6 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 30 | 2.80 |
| 7 | neotame | 30 | 2.80 |
| 8 | peppermint supreme | 20 | 1.87 |
| 9 | spearmint oil | 20 | 1.87 |
| 10 | cinnamon oil | 15 | 1.40 |
| 11 | menthol | 10 | 0.93 |
| 12 | ethanol | 100 | 9.35 |
| 13 | Tween-80 | 100 | 9.35 |

### Manufacturing Procedure:

Accurately weighed quantity of omega fatty acid and Tween-80 were taken into a glass vial to which decanoic acid, PEG-PE and egg lecithin were added and the contents were dissolved by heating on a water bath at 40°C. Then, required quantity of cannabidiol followed by gabapentin were added to the above mixture and dissolved by further heating at 40°C to give a homogenous oil matrix which was then cooled to room temperature.

In another vial weighed quantity of ethanol was taken to which neotame, menthol, spearmint oil, cinnamon oil and peppermint supreme were added and dissolved by stirring for 5 min at 400 rpm. The ethanolic mixture of this vial was transferred to the vial containing cooled oil matrix and the resultant mixture was mixed for 2 min at 400 rpm.

### Example 22: Self -dispersible atorvastatin oral formulation (comparative)

| **S. No** | **Ingredients** | **Quantity (mg)** | **%w/v** |
|---|---|---|---|
| 1 | atorvastatin | 80 | 7.48 |
| 2 | grapeseed oil | 300 | 28.04 |
| 3 | decanoic acid | 50 | 4.67 |
| 4 | egg lecithin | 50 | 4.67 |
| 5 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 30 | 2.80 |
| 6 | neotame | 30 | 2.80 |
| 7 | peppermint supreme | 20 | 1.87 |
| 8 | spearmint oil | 20 | 1.87 |
| 9 | cinnamon oil | 15 | 1.40 |
| 10 | menthol | 10 | 0.93 |
| 11 | ethanol | 100 | 9.35 |
| 12 | Kolliphor RH40 | 100 | 9.35 |
| 13 | Labrafac Lipophile WL 1349 (Medium Chain Triglycerides NF) | 195 | 18.22 |

### Manufacturing Procedure:

Accurately weighed quantity of Labrafac Lipophile WL 1349 (Medium Chain Triglycerides NF) Kolliphor RH40 and grapeseed oil were taken into a glass vial to which decanoic acid, PEG-PE and egg lecithin were added and the contents were dissolved by heating on a water bath at 40°C. Then, required quantity of atorvastatin was added to the above mixture followed by cooling to room temperature.

In another vial weighed quantity of ethanol was taken to which neotame, menthol, spearmint oil, cinnamon oil and peppermint supreme were added and dissolved by stirring for 5 min at 400 rpm. The ethanolic mixture of this vial was transferred to the vial containing cooled oil matrix and the resultant mixture was mixed for 2 min at 400 rpm.

### Example 23: Self-dispersible atorvastatin oral formulation

| **S. No** | **Ingredients** | **Quantity (mg)** | **%w/v** |
|---|---|---|---|
| 1 | atorvastatin | 80 | 7.48 |
| 2 | cannabidiol | 100 | 9.35 |
| 3 | grapeseed oil | 300 | 28.04 |
| 4 | decanoic acid | 50 | 4.67 |
| 5 | egg lecithin | 50 | 4.67 |
| 6 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 30 | 2.80 |
| 7 | neotame | 30 | 2.80 |
| 8 | peppermint supreme | 20 | 1.87 |
| 9 | spearmint oil | 20 | 1.87 |
| 10 | cinnamon oil | 15 | 1.40 |
| 11 | menthol | 10 | 0.93 |
| 12 | ethanol | 100 | 9.35 |
| 13 | Kolliphor RH40 | 100 | 9.35 |
| 14 | Labrafac Lipophile WL 1349 (Medium Chain Triglycerides NF) | 95 | 8.88 |

### Manufacturing Procedure:

Accurately weighed quantity of Labrafac Lipophile WL 1349 (Medium Chain Triglycerides NF) Kolliphor RH40 and grapeseed oil were taken into a glass vial to which decanoic acid, PEG-PE, egg lecithin and cannabidiol were added and the contents were dissolved by heating on a water bath at 40°C. Then, required quantity of atorvastatin was added to the above mixture followed by cooling to room temperature.

In another vial weighed quantity of ethanol was taken to which neotame, menthol, spearmint oil, cinnamon oil and peppermint supreme were added and dissolved by stirring for 5 min at 400 rpm. The ethanolic mixture of this vial was transferred to the vial containing cooled oil matrix and the resultant mixture was mixed for 2 min at 400 rpm.

### Example 24: Self -dispersible duloxetine oral liquid oil (comparative)

| **S. No** | **Ingredients** | **Quantity (mg)** | **%w/v** |
|---|---|---|---|
| 1 | duloxetine | 60 | 5.61 |
| 2 | Labrafac Lipophile WL 1349 (Medium Chain Triglycerides NF) | 515 | 48.13 |
| 3 | decanoic acid | 50 | 4.67 |
| 4 | egg lecithin | 50 | 4.67 |
| 5 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 30 | 2.80 |
| 6 | neotame | 30 | 2.80 |
| 7 | peppermint supreme | 20 | 1.87 |
| 8 | spearmint oil | 20 | 1.87 |
| 9 | cinnamon oil | 15 | 1.40 |
| 10 | menthol | 10 | 0.93 |
| 11 | ethanol | 100 | 9.35 |
| 12 | Tween-80 | 100 | 9.35 |

### Manufacturing Procedure:

Accurately weighed quantity of Labrafac Lipophile WL 1349 (Medium Chain Triglycerides NF)and Tween-80 were taken into a glass vial to which decanoic acid, PEG-PE and egg lecithin were added and the contents were dissolved by heating on a water bath at 40°C. Then, required quantity of duloxetine was added to the above mixture and dissolved by further heating at 40°C to give a homogenous oil matrix which was then cooled to room temperature.

In another vial weighed quantity of ethanol was taken to which neotame, menthol, spearmint oil, cinnamon oil and peppermint supreme were added and dissolved by stirring for 5 min at 400 rpm. The ethanolic mixture of this vial was transferred to the vial containing cooled oil matrix and the resultant mixture was mixed for 2 min at 400 rpm.

The oils used in the above formulations can be corn oil, coconut oil, fish oil, EPA, DHA, soybean oil, olive oil, flax seed oil, avocado oil, ginger oil, hemp seed oil, and grapeseed oil.

### Example 25: Self- dispersible duloxetine oral liquid oil

| **S. No** | **Ingredients** | **Quantity (mg)** | **%w/v** |
|---|---|---|---|
| 1 | duloxetine | 60 | 5.61 |
| 2 | cannabidiol | 50 | 4.67 |
| 3 | Labrafac Lipophile WL 1349 (Medium Chain Triglycerides NF) | 465 | 43.46 |
| 4 | decanoic acid | 50 | 4.67 |
| 5 | egg lecithin | 50 | 4.67 |
| 6 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 30 | 2.80 |
| 7 | neotame | 30 | 2.80 |
| 8 | peppermint supreme | 20 | 1.87 |
| 9 | spearmint oil | 20 | 1.87 |
| 10 | cinnamon oil | 15 | 1.40 |
| 11 | menthol | 10 | 0.93 |
| 12 | ethanol | 100 | 9.35 |
| 13 | Tween-80 | 100 | 9.35 |

### Manufacturing Procedure:

Accurately weighed quantity of Labrafac Lipophile WL 1349 (Medium Chain Triglycerides NF)and Tween-80 were taken into a glass vial to which decanoic acid, PEG-PE and egg lecithin were added and the contents were dissolved by heating on a water bath at 40°C. Then, required quantity of cannabidiol followed by duloxetine was added to the above mixture and dissolved by further heating at 40°C to give a homogenous oil matrix which was then cooled to room temperature.

In another vial weighed quantity of ethanol was taken to which neotame, menthol, spearmint oil, cinnamon oil and peppermint supreme were added and dissolved by stirring for 5 min at 400 rpm. The ethanolic mixture of this vial was transferred to the vial containing cooled oil matrix and the resultant mixture was mixed for 2 min at 400 rpm.

The oils used in the above formulations can be corn oil, coconut oil, fish oil, EPA, DHA, soybean oil, olive oil, flax seed oil, avocado oil, ginger oil, hemp seed oil, and grapeseed oil.

### Example 26: Self-dispersible naproxen sodium oral formulation (comparative)

| **S. No** | **Ingredients** | **Quantity (mg)** | **%w/v** |
|---|---|---|---|
| 1 | naproxen sodium | 100 | 9.35 |
| 2 | grapeseed oil | 300 | 28.04 |
| 3 | decanoic acid | 50 | 4.67 |
| 4 | egg lecithin | 50 | 4.67 |
| 5 | N-(Carbonyl-methoxypolyethylenglycol-2000)-1,2-distearoyl-sn-glycero-3-phosphoethanolamine, MPEG-2000-D SPE, Na-salt (Lipoid PE 18:0/18:0-PEG 2000) | 30 | 2.80 |
| 6 | neotame | 30 | 2.80 |
| 7 | peppermint supreme | 20 | 1.87 |
| 8 | spearmint oil | 20 | 1.87 |
| 9 | cinnamon oil | 15 | 1.40 |
| 10 | menthol | 10 | 0.93 |
| 11 | ethanol | 100 | 9.35 |
| 12 | Kolliphor RH40 | 100 | 9.35 |
| 13 | Labrafac Lipophile WL 1349 (Medium Chain Triglycerides NF) | 175 | 16.36 |

### Manufacturing Procedure:

Accurately weighed quantity of Labrafac Lipophile WL 1349 (Medium Chain Triglycerides NF) Kolliphor RH40 and grapeseed oil were taken into a glass vial to which decanoic acid, PEG-PE and egg lecithin were added and the contents were dissolved by heating on a water bath at 40°C. Then, required quantity of naproxen sodium was added to the above mixture followed by cooling to room temperature.

In another vial weighed quantity of ethanol was taken to which neotame, menthol, spearmint oil, cinnamon oil and peppermint supreme were added and dissolved by stirring for 5 min at 400 rpm. The ethanolic mixture of this vial was transferred to the vial containing cooled oil matrix and the resultant mixture was mixed for 2 min at 400 rpm.

### Example 27: Self -dispersible naproxen sodium oral formulation

| **S. No** | **Ingredients** | **Quantity (mg)** | **%w/v** |
|---|---|---|---|
| 1 | naproxen sodium | 100 | 9.35 |
| 2 | cannabidiol | 50 | 4.67 |
| 3 | grapeseed oil | 300 | 28.04 |
| 4 | decanoic acid | 50 | 4.67 |
| 5 | egg lecithin | 50 | 4.67 |
| 6 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 30 | 2.80 |
| 7 | neotame | 30 | 2.80 |
| 8 | peppermint supreme | 20 | 1.87 |
| 9 | spearmint oil | 20 | 1.87 |
| 10 | cinnamon oil | 15 | 1.40 |
| 11 | menthol | 10 | 0.93 |
| 12 | ethanol | 100 | 9.35 |
| 13 | Kolliphor RH40 | 100 | 9.35 |
| 14 | Labrafac Lipophile WL 1349 (Medium Chain Triglycerides NF) | 125 | 11.68 |

### Manufacturing Procedure:

Accurately weighed quantity of Labrafac Lipophile WL 1349 (Medium Chain Triglycerides NF) Kolliphor RH40 and grapeseed oil were taken into a glass vial to which decanoic acid, PEG-PE, egg lecithin and cannabidiol were added and the contents were dissolved by heating on a water bath at 40°C. Then, required quantity of naproxen sodium was added to the above mixture followed by cooling to room temperature.

In another vial weighed quantity of ethanol was taken to which neotame, menthol, spearmint oil, cinnamon oil and peppermint supreme were added and dissolved by stirring for 5 min at 400 rpm. The ethanolic mixture of this vial was transferred to the vial containing cooled oil matrix and the resultant mixture was mixed for 2 min at 400 rpm.

### Example 28: Self -dispersible abiraterone acetate oral formulation (comparative)

| **S. No** | **Ingredients** | **Quantity (mg)** | **%w/v** |
|---|---|---|---|
| 1 | abiraterone acetate | 100 | 9.35 |
| 2 | Labrafac Lipophile WL 1349 (Medium Chain Triglycerides NF) | 475 | 44.39 |
| 3 | decanoic acid | 50 | 4.67 |
| 4 | egg lecithin | 50 | 4.67 |
| 5 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 30 | 2.80 |
| 6 | neotame | 30 | 2.80 |
| 7 | peppermint supreme | 20 | 1.87 |
| 8 | spearmint oil | 20 | 1.87 |
| 9 | cinnamon oil | 15 | 1.40 |
| 10 | menthol | 10 | 0.93 |
| 11 | ethanol | 100 | 9.35 |
| 12 | Kolliphor RH40 | 100 | 9.35 |

### Manufacturing Procedure:

Accurately weighed quantity of Labrafac Lipophile WL 1349 (Medium Chain Triglycerides NF) and Kolliphor RH40 were taken into a glass vial to which decanoic acid, PEG-PE and egg lecithin were added and the contents were dissolved by heating on a water bath at 40°C. Then, required quantity of abiraterone acetate was added to the above mixture followed by cooling to room temperature.

In another vial weighed quantity of ethanol was taken to which neotame, menthol, spearmint oil, cinnamon oil and peppermint supreme were added and dissolved by stirring for 5 min at 400 rpm. The ethanolic mixture of this vial was transferred to the vial containing cooled oil matrix and the resultant mixture was mixed for 2 min at 400 rpm.

The concentration of Lipoid PE 18:0/18:0-PEG 2000 in the formulation may vary from 0 to 6% of the total formulation in the case of self-dispersible nano-carrier concentrate to be filled in capsules.

### Example 29: Self-dispersible abiraterone acetate oral formulation

| **S. No** | **Ingredients** | **Quantity (mg)** | **%w/v** |
|---|---|---|---|
| 1 | abiraterone acetate | 100 | 9.35 |
| 2 | cannabidiol | 50 | 4.67 |
| 3 | Labrafac Lipophile WL 1349 (Medium Chain Triglycerides NF) | 425 | 39.72 |
| 4 | decanoic acid | 50 | 4.67 |
| 5 | egg lecithin | 50 | 4.67 |
| 6 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 30 | 2.80 |
| 7 | neotame | 30 | 2.80 |
| 8 | peppermint supreme | 20 | 1.87 |
| 9 | spearmint oil | 20 | 1.87 |
| 10 | cinnamon oil | 15 | 1.40 |
| 11 | menthol | 10 | 0.93 |
| 12 | ethanol | 100 | 9.35 |
| 13 | Kolliphor RH40 | 100 | 9.35 |

### Manufacturing Procedure:

Accurately weighed quantity of Labrafac Lipophile WL 1349 (Medium Chain Triglycerides NF) and Kolliphor RH40 were taken into a glass vial to which decanoic acid, PEG-PE, egg lecithin and cannabidiol were added and the contents were dissolved by heating on a water bath at 40°C. Then, required quantity of abiraterone acetate was added to the above mixture followed by cooling to room temperature.

In another vial weighed quantity of ethanol was taken to which neotame, menthol, spearmint oil, cinnamon oil and peppermint supreme were added and dissolved by stirring for 5 min at 400 rpm. The ethanolic mixture of this vial was transferred to the vial containing cooled oil matrix and the resultant mixture was mixed for 2 min at 400 rpm.

The concentration of Lipoid PE 18:0/18:0-PEG 2000 in the formulation may vary from 0 to 6% of the total formulation in the case of self-dispersible nanocarrier concentrate to be filled in capsules.

### Example 30: Self -dispersible cabazitaxel oral formulation (comparative)

| **S. No** | **Ingredients** | **Quantity (mg)** | **%w/v** |
|---|---|---|---|
| 1 | cabazitaxel | 120 | 11.21 |
| 2 | Labrafac Lipophile WL 1349 (Medium Chain Triglycerides NF) | 455 | 42.52 |
| 3 | decanoic acid | 50 | 4.67 |
| 4 | egg lecithin | 50 | 4.67 |
| 5 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 30 | 2.80 |
| 6 | neotame | 30 | 2.80 |
| 7 | peppermint supreme | 20 | 1.87 |
| 8 | spearmint oil | 20 | 1.87 |
| 9 | cinnamon oil | 15 | 1.40 |
| 10 | menthol | 10 | 0.93 |
| 11 | ethanol | 100 | 9.35 |
| 12 | Kolliphor RH40 | 100 | 9.35 |

### Manufacturing Procedure:

Accurately weighed quantity of Labrafac Lipophile WL 1349 (Medium Chain Triglycerides NF) and Kolliphor RH40 were taken into a glass vial to which decanoic acid, PEG-PE and egg lecithin were added and the contents were dissolved by heating on a water bath at 40°C. Then, required quantity of cabazitaxel was added to the above mixture followed by cooling to room temperature.

In another vial weighed quantity of ethanol was taken to which neotame, menthol, spearmint oil, cinnamon oil and peppermint supreme were added and dissolved by stirring for 5 min at 400 rpm. The ethanolic mixture of this vial was transferred to the vial containing cooled oil matrix and the resultant mixture was mixed for 2 min at 400 rpm.

The concentration of Lipoid PE 18:0/18:0-PEG 2000 in the formulation may vary from 0 to 6% of the total formulation in the case of self-dispersible nanocarrier concentrate to be filled in capsules.

### Example 31: Self -dispersible cabazitaxel oral formulation

| **S. No** | **Ingredients** | **Quantity (mg)** | **%w/v** |
|---|---|---|---|
| 1 | cabazitaxel | 120 | 11.21 |
| 2 | cannabidiol | 50 | 4.67 |
| 3 | Labrafac Lipophile WL 1349 (Medium Chain Triglycerides NF) | 405 | 37.85 |
| 4 | decanoic acid | 50 | 4.67 |
| 5 | egg lecithin | 50 | 4.67 |
| 6 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 30 | 2.80 |
| 7 | neotame | 30 | 2.80 |
| 8 | peppermint supreme | 20 | 1.87 |
| 9 | spearmint oil | 20 | 1.87 |
| 10 | cinnamon oil | 15 | 1.40 |
| 11 | menthol | 10 | 0.93 |
| 12 | ethanol | 100 | 9.35 |
| 13 | Kolliphor RH40 | 100 | 9.35 |

Accurately weighed quantity of Labrafac Lipophile WL 1349 (Medium Chain Triglycerides NF) and Kolliphor RH40 were taken into a glass vial to which decanoic acid, PEG-PE, egg lecithin and cannabidiol were added and the contents were dissolved by heating on a water bath at 40°C. Then, required quantity of cabazitaxel was added to the above mixture followed by cooling to room temperature.

In another vial weighed quantity of ethanol was taken to which neotame, menthol, spearmint oil, cinnamon oil and peppermint supreme were added and dissolved by stirring for 5 min at 400 rpm. The ethanolic mixture of this vial was transferred to the vial containing cooled oil matrix and the resultant mixture was mixed for 2 min at 400 rpm.

### Example 32: Self -dispersible cannabidiol oral liquid oil

| **S. No** | **Ingredients** | **Quantity (mg)** | **%w/v** |
|---|---|---|---|
| 1 | cannabidiol synthetic | 8 | 0.75 |
| 2 | grapeseed oil | 400 | 37.38 |
| 3 | decanoic acid | 50 | 4.67 |
| 4 | egg lecithin | 50 | 4.67 |
| 5 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 0.3 | 0.03 |
| 6 | neotame | 30 | 2.80 |
| 7 | peppermint supreme | 20 | 1.87 |
| 8 | spearmint oil | 20 | 1.87 |
| 9 | cinnamon oil | 15 | 1.40 |
| 10 | menthol | 10 | 0.93 |
| 11 | ethanol | 100 | 9.35 |
| 12 | Kolliphor RH40 | 100 | 9.35 |
| 13 | Labrafac Lipophile WL 1349 (Medium Chain Triglycerides NF) | 197 | 18.41 |

### Manufacturing Procedure:

Accurately weighed quantity of Labrafac Lipophile WL 1349 (Medium Chain Triglycerides NF), grapeseed oil and Kolliphor RH40 were taken into a glass vial to which decanoic acid, PEG-PE and egg lecithin were added and the contents were dissolved by heating on a water bath at 40°C. Then, required quantity of cannabidiol was added to the above mixture and dissolved by further heating at 40°C to give a homogenous oil matrix which was then cooled to room temperature.

In another vial weighed quantity of ethanol was taken to which neotame, menthol, spearmint oil, cinnamon oil and peppermint supreme were added and dissolved by stirring for 5 min at 400 rpm. The ethanolic mixture of this vial was transferred to the vial containing cooled oil matrix and the resultant mixture was mixed for 2 min at 400 rpm.

### Example 33: Self -dispersible cannabidiol oral liquid oil

| **S. No** | **Ingredients** | **Quantity (mg)** | **%w/v** |
|---|---|---|---|
| 1 | cannabidiol synthetic | 8 | 0.75 |
| 2 | paclitaxel | 30 | 2.80 |
| 3 | grapeseed oil | 350 | 32.71 |
| 4 | decanoic acid | 50 | 4.67 |
| 5 | egg lecithin | 50 | 4.67 |
| 6 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 0.3 | 0.03 |
| 7 | neotame | 30 | 2.80 |
| 8 | peppermint supreme | 20 | 1.87 |
| 9 | spearmint oil | 20 | 1.87 |
| 10 | cinnamon oil | 15 | 1.40 |
| 11 | menthol | 10 | 0.93 |
| 12 | ethanol | 100 | 9.35 |
| 13 | Kolliphor RH40 | 100 | 9.35 |
| 14 | Labrafac Lipophile WL 1349 (Medium Chain Triglycerides NF) | 157 | 14.67 |

### Manufacturing Procedure:

Accurately weighed quantity of Labrafac Lipophile WL 1349 (Medium Chain Triglycerides NF), grapeseed oil and Kolliphor RH40 were taken into a glass vial to which decanoic acid, PEG-PE and egg lecithin were added and the contents were dissolved by heating on a water bath at 40°C. Then, required quantity of cannabidiol followed by paclitaxel were added to the above mixture and dissolved by further heating at 40°C to give a homogenous oil matrix which was then cooled to room temperature.

In another vial weighed quantity of ethanol was taken to which neotame, menthol, spearmint oil, cinnamon oil and peppermint supreme were added and dissolved by stirring for 5 min at 400 rpm. The ethanolic mixture of this vial was transferred to the vial containing cooled oil matrix and the resultant mixture was mixed for 2 min at 400 rpm.

The concentration of Lipoid PE 18:0/18:0-PEG 2000 in the formulation may vary from 5.61 to 18.69 % (w/v) of the total formulation in the case of self-dispersible nanocarrier concentrate to be filled in capsules.

The concentration of Lipoid PE 18:0/18:0-PEG 2000 in the formulation may vary from 0.03 to 9.35 % (w/v) of the total formulation in the case of orally administered dispersible liquid oil.

The sweeteners used in the case of orally administered dispersible liquid oil may be neotame, neohesperidine dihydrochalcone, sucralose, aspartame and erythritol.

The flavouring agents used may be single or a mixture of peppermint supreme, spearmint oil, cinnamon oil and menthol.

The surfactants used for improving the dispersibility of the formulations can be Kolliphor RH40, sucrose laurate, sucrose palmitate, Tween-80, Solutol HS15 or a combination of these surfactants.

### Example 34: Netupitant-CBD NLC formulation (B#117A)

| **S. No** | **Ingredients** | **Quantity (mg/mL)** | **% (w/v)** |
|---|---|---|---|
| 1 | decanoic acid | 12 | 1.2 |
| 2 | Labrafac Lipophile WL 1349 (Medium Chain Triglycerides NF) | 12 | 1.2 |
| 3 | N-(Carbonyl methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 3 | 0.3 |
| 4 | cannabidiol | 1.2 | 0.12 |
| 5 | netupitant | 4 | 0.4 |
| 6 | palonosetron hydrochloride | 0.005 | 0.0005 |
| 7 | ethanol | 10 | 1.0 |
| 8 | egg lecithin (Lipoid E80S) | 10 | 1.0 |
| 9 | cholesterol | 3 | 0.3 |
| 10 | poloxamer-188 | 10 | 1.0 |
| 11 | vitamin E acetate | 0.2 | 0.02 |
| 12 | ascorbyl palmitate | 0.2 | 0.02 |
| 13 | 1,4,7,10-Tetraazacyclododecane-1,4,7,10-tetraacetic acid (DOTA) | 0.02 | 0.002 |
| 14 | chlorbutanol | 0.25 | 0.025 |
| 15 | L-histidine | 5 | 0.5 |
| 16 | dextrose anhydrous | 35 | 3.5 |
| 17 | Milli-Q Water | Q.S to 1 mL | Q.S to 100% |

### Manufacturing Procedure:

**Preparation of Oil Phase:** Accurately weighed quantities of vitamin E acetate was taken into a manufacturing vessel. Labrafac Lipophile WL 1349 (oil) was subsequently added to it followed by decanoic acid and dissolved by heating on a water bath at 55°C (50°C to 60°C). Weighed quantities of ascorbyl palmitate, cholesterol, PEG-PE and egg lecithin (Lipoid E80S) were further added and dissolved. This mixture was cooled to 40°C and ethanol was added. Weighed amount of netupitant and CBD were added to the above mixture and dissolved to give a lipid phase.

**Preparation of Aqueous Phase:** 90% of required quantity of Milli-Q water per batch was taken in another manufacturing vessel in which weighed quantity of poloxamer-188 was dissolved by heating at 55°C (50°C to 60°C). Dextrose, chlorbutanol and DOTA were subsequently added and dissolved in the above solution.

### Preparation of NLC:

**High Shear Homogenization:** The lipid phase was added into the aqueous phase, under homogenization at 4000 rpm by maintaining the product temperature at 55°C (50°C to 60°C) and the homogenization was continued further for 15 min at 8500 rpm. Later, the volume was adjusted up to required level with remaining quantity of Milli-Q water followed by homogenization at 8500 rpm for 15 minutes for formation of a coarse dispersion.

**High Pressure Homogenization:** The obtained coarse dispersion was subjected to high pressure homogenization for 3 passes at different pressures i.e. Pass 1 at 5,000 psi, Pass 2 at 10,000 psi and Pass 3 at 18,000 psi.

Required quantity of Palonosetron HCl was added from prepared stock solution after high pressure homogenization. Finally, the pH of the preparation was adjusted in between 6.0 - 6.5 with L-histidine. The size and zeta potential of the undiluted NLC dispersion were measured using Particle Size Analyser (Lite Sizer, Anton Paar).

Further, the NLC formulation was subjected to long term at four temperature conditions (2-8°C, 25±2°C, 30±2°C and 40±2°C). The data evaluated in stability study is presented in Table 9 and Table 10.

**Table 9: Particle size distribution data of netupitant-CBD NLC (B#117 A)**

| **Stability Condition** | | **HDD (nm)** | **PDI (%)** | **D10 (nm)** | **D50 (nm)** | **D90 (nm)** | **SPAN** | **Zeta Potential (mv)** |
|---|---|---|---|---|---|---|---|---|
| Initial | | 196.24 | 21.83 | 106.90 | 191.67 | 320.49 | 1.12 | -16.20 |
| 6 Months | 2-8°C | 207.73 | 19.03 | 124.15 | 196.95 | 299.73 | 0.89 | -19.93 |
| | 25±2°C | 225.95 | 18.70 | 129.51 | 219.97 | 358.13 | 1.03 | -9.53 |
| | 30±2°C | 234.95 | 17.50 | 141.56 | 222.24 | 340.54 | 0.89 | -12.37 |
| | 40±2°C | 207.56 | 18.90 | 123.33 | 193.26 | 302.59 | 0.93 | -6.30 |

**Table 10: Assay and Impurity Profile of B#117A**

| **Netupitant** | | | | | | |
|---|---|---|---|---|---|---|
| **Batch No** | | | **117A** | | | |
| **Condition** | | **Initial** | **6M** | | | |
| **Time point** | | | **2-8°C** | **25°C** | **30°C** | **40°C** |
| Name of the Impurity | **RRT** | **%w/w** | | | | |
| Unknown | 0.8 | ND | ND | ND | ND | ND |
| Unknown | 0.91 | ND | ND | ND | ND | ND |
| Unknown | 0.95 | ND | 0.05 | 0.04 | 0.03 | 0.24 |
| Unknown | 1.05 | ND | ND | ND | ND | ND |
| N-Oxide | 1.08 | 0.1 | 0.09 | 0.5 | 0.59 | 0.32 |
| **Total impurities** | | **0.1** | 0.14 | 0.54 | 0.62 | **0.56** |
| Assay | | 105.2 | 103.7 | 101.9 | 102.1 | 99.8 |
| pH | | 6.11 | 6.49 | 6.42 | 6.35 | 5.98 |
| Osmolality | | NA | 313 | 309 | 313 | 310 |

| **Cannabidiol** | | | | | | |
|---|---|---|---|---|---|---|
| **Condition** | | **Initial** | **6M** | | | |
| **Time point** | | | **2-8°C** | **25°C** | **30°C** | **40°C** |
| **Related substances** | **RRT** | **%w/w** | | | | |
| Unknown | 2.54 | ND | ND | ND | ND | 0.02 |
| Total impurities | | **ND** | **ND** | **ND** | **ND** | **0.02** |
| Assay | | **105.2** | **105.3** | **102.4** | **103.2** | **96.6** |

| **Palonosetron** | | | | | | |
|---|---|---|---|---|---|---|
| **Condition** | | **Initial** | **6M** | | | |
| **Time Point** | | | **2-8°C** | **25°C** | **30°C** | **40°C** |
| **Related substances** | | **%w/w** | | | | |
| Unknown | | ND | ND | ND | ND | ND |
| N-Oxide | | ND | ND | ND | ND | 0.08 |
| Assay | | 105.9 | **100.2** | **99.1** | **101.3** | **97.6** |

### Example 35: Cannabidiol (CBD) oral NLC formulation (B#130)

| **S. No** | **Ingredients** | **Quantity (mg/mL)** | **%w/v)** |
|---|---|---|---|
| 1 | decanoic acid | 8 | 0.8 |
| 2 | Labrafac Lipophile WL 1349 (Medium Chain Triglycerides NF) | 8 | 0.8 |
| 3 | N-(Carbonyl- methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 3 | 0.3 |
| 4 | cannabidiol synthetic | 8 | 0.8 |
| 5 | ethanol | 10 | 1 |
| 6 | egg lecithin (Lipoid E 80 S) | 5 | 0.5 |
| 7 | cholesterol | 3 | 0.3 |
| 8 | ascorbyl palmitate | 0.2 | 0.02 |
| 9 | propyl paraben | 0.2 | 0.02 |
| 10 | peppermint supreme | 1 | 0.1 |
| 11 | poloxamer-188 | 10 | 1 |
| 12 | DOTA | 0.02 | 0.002 |
| 13 | tris base | 1.5 | 0.15 |
| 14 | dextrose anhydrous | 35 | 3.5 |
| 15 | Milli-Q Water | Q.S to 1 mL | Q.S to 100% |

### Manufacturing Procedure:

**Preparation of Oil Phase:** Accurately weighed quantity of Labrafac Lipophile WL 1349 (oil) was taken into a manufacturing vessel to which decanoic acid was added and dissolved by heating on a water bath at 55°C (50°C to 60°C). Weighed quantities of cholesterol, PEG-PE and egg lecithin (Lipoid E80S) were subsequently added and dissolved to give a mixture which was cooled to 40°C. Weighed amount of CBD was added to the above mixture and dissolved. In another vessel peppermint supreme, propyl paraben, ascorbyl palmitate were dissolved in ethanol. The contents of both the vessels were mixed to give a lipid phase.

**Preparation of Aqueous Phase:** 90% of required quantity of Milli-Q water per batch was taken in another manufacturing vessel in which weighed quantity of poloxamer-188 was dissolved by heating at 55°C (50°C to 60°C). Dextrose and DOTA were subsequently added and dissolved in the above solution.

### Preparation of NLC:

**High Shear Homogenization:** The lipid phase was added into the aqueous phase, under homogenization at 4000 rpm by maintaining the product temperature at 55°C (50°C to 60°C) and the homogenization was continued further for 15 min at 8500 rpm. Later, the volume was adjusted up to required level with remaining quantity of Milli-Q water followed by homogenization at 8500 rpm for 15 minutes for formation of a coarse dispersion.

**High Pressure Homogenization:** The obtained coarse dispersion was subjected to high pressure homogenization for 3 passes at different pressures i.e. Pass 1 at 5,000 psi, Pass 2 at 10,000 psi and Pass 3 at 18,000 psi.

The pH of the formulation was adjusted to 7.0-7.5 with Tris base. The size and zeta potential of the undiluted NLC dispersion was measured using particle size analyser (Lite Sizer, Anton Paar).

Further, the NLC formulation was subjected to 1 year stability at 2-8°C and 25±2°C. The data evaluated in stability study is presented in Table 11 and Table 12.

**Table 11: Particle size distribution data of CBD Oral NLC (B#013)**

| **Stability Condition** | | **HDD (nm)** | **PDI (%)** | **D10 (nm)** | **D50 (nm)** | **D90 (nm)** | **SPA N** | **Zeta Potent ial (mv)** |
|---|---|---|---|---|---|---|---|---|
| Initial | | 112.84 | 19.67 | 63.17 | 113.60 | 189.45 | 1.11 | -9.43 |
| 12 M | 2-8°C | 141.78 | 17.83 | 82.98 | 134.86 | 211.23 | 0.95 | -6.03 |
| | 25±2 °C | 154.13 | 17.57 | 94.12 | 143.02 | 216.07 | 0.85 | -9.03 |

**Table 12: Assay and impurity profile of cannabidiol oral NLC (B#130)**

| **Cannabidiol** | | | |
|---|---|---|---|
| **Batch No** | **130** | | |
| **Condition** | **Initial** | **12M** | **12M** |
| **Time point** | | **2-8°C** | **25°C** |
| **Related substances** | **%w/w** | | |
| **Total impurities** | ND | ND | ND |
| **Assay** | 101.7 | 98.3 | 96.6 |
| **pH** | 6.48 | 6.49 | 6.51 |
| **Osmolality** | NA | 266 | 271 |

| | | | |
|---|---|---|---|
| *Note: The formulation is Tetrahydrocannabinol (THC) free. | | | |

### Example 36: Cannabidiol (CBD) oral NLC formulation (B#013)

| **S. No** | **Ingredients** | **Quantity (mg/mL)** | **% (w/v)** |
|---|---|---|---|
| 1 | decanoic acid | 8 | 0.8 |
| 2 | Labrafac Lipophile WL 1349 (Medium Chain Triglycerides NF) | 8 | 0.8 |
| 3 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 6 | 0.6 |
| 4 | cannabidiol synthetic | 8 | 0.8 |
| 5 | ethanol | 10 | 1.0 |
| 6 | egg lecithin (Lipoid E80S ) | 5 | 0.5 |
| 7 | cholesterol | 3 | 0.3 |
| 8 | poloxamer-188 | 10 | 1.0 |
| 9 | ascorbyl palmitate | 0.2 | 0.02 |
| 10 | 1,4,7,10-Tetraazacyclododecane-1,4,7,10-tetraacetic acid (DOTA) | 0.02 | 0.002 |
| 11 | propyl paraben | 0.2 | 0.02 |
| 12 | tris base | 1.5 | 0.15 |
| 13 | dextrose anhydrous | 35 | 3.5 |
| 14 | neohesperidine dihydrochalcone | 0.5 | 0.05 |
| 15 | peppermint supreme | 1 | 0.1 |
| 16 | Milli-Q Water | Q.S to 1 mL | Q.S to 100% |

### Manufacturing Procedure:

**Preparation of Oil Phase:** Accurately weighed quantity of Labrafac Lipophile WL 1349 (oil) was taken into a manufacturing vessel to which decanoic acid was added and dissolved by heating on a water bath at 55°C (50°C to 60°C). Weighed quantities of cholesterol, PEG-PE and egg lecithin (Lipoid E80S) were subsequently added and dissolved to give a mixture which was cooled to 40°C. Weighed amount of CBD was added to the above mixture and dissolved. In another vessel peppermint supreme, propyl paraben, ascorbyl palmitate were dissolved in ethanol. The contents of both the vessels were mixed to give a lipid phase.

**Preparation of Aqueous Phase:** 90% of required quantity of Milli-Q water per batch was taken in another manufacturing vessel in which weighed quantity of poloxamer-188 and neohesperidine dihydrochalcone were dissolved by heating at 55°C (50°C to 60°C). Dextrose and DOTA were subsequently added and dissolved in the above solution.

### Preparation of NLC:

**High Shear Homogenization:** The lipid phase was added into the aqueous phase, under homogenization at 4000 rpm by maintaining the product temperature at 55°C (50°C to 60°C) and the homogenization was continued further for 15 min at 8500 rpm. Later, the volume was adjusted up to required level with remaining quantity of Milli-Q water followed by homogenization at 8500 rpm for 15 minutes for formation of a coarse dispersion.

**High Pressure Homogenization:** The obtained coarse dispersion was subjected to high pressure homogenization for 3 passes at different pressures i.e. Pass 1 at 5,000 psi, Pass 2 at 10,000 psi and Pass 3 at 18,000 psi.

The pH of the formulation was adjusted to 7.0-7.5 with tris base. The size and zeta potential of the undiluted NLC dispersion was measured using particle size analyser (Lite Sizer, Anton Paar).

Further, the NLC formulation was subjected to stability at different temperature conditions. The data evaluated in stability study is presented in Table 13 and Table 14.

**Table 13: Particle size distribution data of CBD oral NLC (B#013)**

| **Stability condition** | | **HDD (nm)** | **PDI (%)** | **D10 (nm)** | **D50 (nm)** | **D90 (nm)** | **SP AN** | **Zeta Potent ial (mv)** |
|---|---|---|---|---|---|---|---|---|
| Initial | | 96.52 | 22.33 | 50.70 | 100.44 | 175.6 | 1.25 | -16.1 |
| 3 Months | 2-8°C | 129.05 | 17.63 | 76.14 | 124.01 | 196.75 | 0.97 | -4.20 |
| | 25±2°C | 130.98 | 16.63 | 77.80 | 123.47 | 192.31 | 0.92 | -4.80 |

**Table 14: Assay and impurity profile of cannabidiol oral NLC (B#013)**

| **Cannabidiol** | | | |
|---|---|---|---|
| **Batch No** | **013** | | |
| **Condition** | **Initial** | **3M** | **3M** |
| **Time point** | | **2-8°C** | **25°C** |

| **Related substances** | **%w/w** | | |
|---|---|---|---|
| Total impurities | ND | ND | 0.17 |
| Assay | 102 | 101.3 | 100.4 |
| pH | 6.5 | 6.64 | 6.64 |
| Osmolality | 242 | 218 | 212 |

| | | | |
|---|---|---|---|
| *Note: The formulation is Tetrahydrocannabinol (THC) free. | | | |

### Example 37: Cannabidiol (CBD) oral NLC formulation

| **S. No** | **Ingredients** | **Quantity (mg/mL)** | **(%w/V)** |
|---|---|---|---|
| 1 | Labrafac Lipophile WL 1349 (Medium Chain Triglycerides NF) | 10 | 1.0 |
| 2 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 6 | 0.6 |
| 3 | cannabidiol synthetic | 10 | 1.0 |
| 4 | ethanol | 10 | 1.0 |
| 5 | egg lecithin (Lipoid E80S ) | 5 | 0.5 |
| 6 | poloxamer-188 | 10 | 1.0 |
| 7 | ascorbyl palmitate | 0.2 | 0.02 |
| 8 | 1,4,7,10-Tetraazacyclododecane-1,4,7,10-tetraacetic acid (DOTA) | 0.02 | 0.002 |
| 9 | propyl paraben | 0.2 | 0.02 |
| 10 | tris base | 0.1 | 0.01 |
| 11 | dextrose anhydrous | 35 | 3.5 |
| 12 | neotame | 1 | 0.1 |
| 13 | peppermint supreme | 2 | 0.2 |
| 14 | menthol | 0.4 | 0.04 |
| 15 | Milli-Q Water | Q.S to 1 mL | Q.S to 100% |

### Manufacturing Procedure:

**Preparation of Oil Phase:** Accurately weighed quantity of Labrafac Lipophile WL 1349 (oil) was taken into a manufacturing vessel. Weighed quantities of PEG-PE and egg lecithin (Lipoid E80S) were subsequently added and dissolved by heating on a water bath at 55°C (50°C to 60°C) to give a mixture which was cooled to 40°C. Weighed amount of CBD was added to the above mixture and dissolved. In another vessel peppermint supreme, propyl paraben, ascorbyl palmitate, neotame and menthol were dissolved in ethanol. The contents of both the vessels were mixed to give a lipid phase.

**Preparation of Aqueous Phase:** 90% of required quantity of Milli-Q water per batch was taken in another manufacturing vessel in which weighed quantity of poloxamer-188 was dissolved by heating at 55°C (50°C to 60°C). Dextrose and DOTA were subsequently added and dissolved in the above solution.

### Preparation of NLC:

**High Shear Homogenization:** The lipid phase was added into the aqueous phase, under homogenization at 4000 rpm by maintaining the product temperature at 55°C (50°C to 60°C) and the homogenization was continued further for 15 min at 8500 rpm. Later, the volume was adjusted up to required level with remaining quantity of Milli-Q water followed by homogenization at 8500 rpm for 15 minutes for formation of a coarse dispersion.

**High Pressure Homogenization:** The obtained coarse dispersion was subjected to high pressure homogenization for 3 passes at different pressures i.e. Pass 1 at 5,000 psi, Pass 2 at 10,000 psi and Pass 3 at 18,000 psi.

The pH of the formulation was adjusted to 7.0-7.5 with Tris base. The size and zeta potential of the undiluted NLC dispersion was measured using particle size analyzer (Lite Sizer, Anton Paar).

**Table 15: Particle size data of oral CBD NLC formulation**

| **HDD (nm)** | **PDI (%)** | **D10 (nm)** | **D50 (nm)** | **D90 (nm)** | **SPAN** | **Zeta Poten tial (mv)** |
|---|---|---|---|---|---|---|
| 117.22 | 21.40 | 66.98 | 115.65 | 185.02 | 1.02 | -16.63 |

### Example 38: Cannabidiol (CBD) Oral NLC formulation

| **S. No** | **Ingredients** | **Quantity (mg/mL)** | **(%w/V)** |
|---|---|---|---|
| 1 | Incromega E3322 (fish oil) | 10 | 1.0 |
| 2 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 6 | 0.6 |
| 3 | cannabidiol synthetic | 10 | 1.0 |
| 4 | ethanol | 10 | 1.0 |
| 5 | egg lecithin (Lipoid E80S ) | 5 | 0.5 |
| 6 | poloxamer-188 | 10 | 1.0 |
| 7 | ascorbyl palmitate | 0.2 | 0.02 |
| 8 | 1,4,7,10-Tetraazacyclododecane-1,4,7,10-tetraacetic acid (DOTA) | 0.02 | 0.002 |
| 9 | propyl paraben | 0.2 | 0.02 |
| 10 | tris base | 0.1 | 0.01 |
| 11 | dextrose anhydrous | 35 | 3.5 |
| 12 | neotame | 1 | 0.1 |
| 13 | peppermint supreme | 2 | 0.2 |
| 14 | menthol | 0.4 | 0.04 |
| 15 | Milli-Q Water | Q.S to 1 mL | Q.S to 100% |

### Manufacturing Procedure:

**Preparation of Oil Phase:** Accurately weighed quantity of Incromega E3322 (Fish Oil) was taken into a manufacturing vessel. Weighed quantities of PEG-PE and egg lecithin (Lipoid E80S) were subsequently added and dissolved by heating on a water bath at 55°C (50°C to 60°C) to give a mixture which was cooled to 40°C. Weighed amount of CBD was added to the above mixture and dissolved. In another vessel peppermint supreme, propyl paraben, ascorbyl palmitate, neotame and menthol were dissolved in ethanol. The contents of both the vessels were mixed to give a lipid phase.

**Preparation of Aqueous Phase:** 90% of required quantity of Milli-Q water per batch was taken in another manufacturing vessel in which weighed quantity of poloxamer-188 was dissolved by heating at 55°C (50°C to 60°C). Dextrose and DOTA were subsequently added and dissolved in the above solution.

### Preparation of NLC:

**High Shear Homogenization:** The lipid phase was added into the aqueous phase, under homogenization at 4000 rpm by maintaining the product temperature at 55°C (50°C to 60°C) and the homogenization was continued further for 15 min at 8500 rpm. Later, the volume was adjusted up to required level with remaining quantity of Milli-Q water followed by homogenization at 8500 rpm for 15 minutes for formation of a coarse dispersion.

**High Pressure Homogenization:** The obtained coarse dispersion was subjected to high pressure homogenization for 3 passes at different pressures i.e. Pass 1 at 5,000 psi, Pass 2 at 10,000 psi and Pass 3 at 18,000 psi.

The pH of the formulation is adjusted to 7.0-7.5 with tris base.

### Example 39: Cannabidiol (CBD) oral NLC formulation

| **S. No** | **Ingredients** | **Quantity (mg/mL)** | **(%w/V)** |
|---|---|---|---|
| 1 | eicosapentaenoic acid (EPA) | 10 | 1.0 |
| 2 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 6 | 0.6 |
| 3 | cannabidiol synthetic | 10 | 1.0 |
| 4 | ethanol | 10 | 1.0 |
| 5 | egg lecithin (Lipoid E80S ) | 5 | 0.5 |
| 6 | poloxamer-188 | 10 | 1.0 |
| 7 | ascorbyl palmitate | 0.2 | 0.02 |
| 8 | 1,4,7, 10-Tetraazacyclododecane-1,4,7,10-tetraacetic acid (DOTA) | 0.02 | 0.002 |
| 9 | propyl paraben | 0.2 | 0.02 |
| 10 | tris base | 0.1 | 0.01 |
| 11 | dextrose anhydrous | 35 | 3.5 |
| 12 | neotame | 1 | 0.1 |
| 13 | peppermint supreme | 2 | 0.2 |
| 14 | menthol | 0.4 | 0.04 |
| 15 | Milli-Q Water | Q.S to 1 mL | Q.S to 100% |

### Manufacturing Procedure:

**Preparation of Oil Phase:** Accurately weighed quantity of eicosapentaenoic acid (EPA) was taken into a manufacturing vessel. Weighed quantities of PEG-PE and egg lecithin (Lipoid E80S) were subsequently added and dissolved by heating on a water bath at 55°C (50°C to 60°C) to give a mixture which was cooled to 40°C. Weighed amount of CBD was added to the above mixture and dissolved. In another vessel peppermint supreme, propyl paraben, ascorbyl palmitate, neotame and menthol were dissolved in ethanol. The contents of both the vessels were mixed to give a lipid phase.

**Preparation of Aqueous Phase:** 90% of required quantity of Milli-Q water per batch was taken in another manufacturing vessel in which weighed quantity of poloxamer-188 was dissolved by heating at 55°C (50°C to 60°C). Dextrose and DOTA were subsequently added and dissolved in the above solution.

### Preparation of NLC:

**High Shear Homogenization:** The lipid phase was added into the aqueous phase, under homogenization at 4000 rpm by maintaining the product temperature at 55°C (50°C to 60°C) and the homogenization was continued further for 15 min at 8500 rpm. Later, the volume was adjusted up to required level with remaining quantity of Milli-Q water followed by homogenization at 8500 rpm for 15 minutes for formation of a coarse dispersion.

**High Pressure Homogenization:** The obtained coarse dispersion was subjected to high pressure homogenization for 3 passes at different pressures i.e. Pass 1 at 5,000 psi, Pass 2 at 10,000 psi and Pass 3 at 18,000 psi.

The pH of the formulation is adjusted to 7.0-7.5 with tris base.

### Example 40: Cannabidiol (CBD) oral NLC formulation

| **S. No** | **Ingredients** | **Quantity (mg/mL)** | **(%w/V)** |
|---|---|---|---|
| 1 | docosahexaenoic acid (DHA) | 10 | 1.0 |
| 2 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 6 | 0.6 |
| 3 | cannabidiol synthetic | 10 | 1.0 |
| 4 | ethanol | 10 | 1.0 |
| 5 | egg lecithin (lipoid e80s ) | 5 | 0.5 |
| 6 | poloxamer-188 | 10 | 1.0 |
| 7 | ascorbyl palmitate | 0.2 | 0.02 |
| 8 | 1,4,7,10-Tetraazacyclododecane-1,4,7,10-tetraacetic acid (DOTA) | 0.02 | 0.002 |
| 9 | propyl paraben | 0.2 | 0.02 |
| 10 | tris base | 0.1 | 0.01 |
| 11 | dextrose anhydrous | 35 | 3.5 |
| 12 | neotame | 1 | 0.1 |
| 13 | peppermint supreme | 2 | 0.2 |
| 14 | menthol | 0.4 | 0.04 |
| 15 | Milli-Q Water | Q.S to 1 mL | Q.S to 100% |

### Manufacturing Procedure:

**Preparation of Oil Phase:** Accurately weighed quantity of docosahexaenoic acid (DHA) was taken into a manufacturing vessel. Weighed quantities of PEG-PE and egg lecithin (Lipoid E80S) were subsequently added and dissolved by heating on a water bath at 55°C (50°C to 60°C) to give a mixture which was cooled to 40°C. Weighed amount of CBD was added to the above mixture and dissolved. In another vessel peppermint supreme, propyl paraben, ascorbyl palmitate, neotame and menthol were dissolved in ethanol. The contents of both the vessels were mixed to give a lipid phase.

**Preparation of Aqueous Phase:** 90% of required quantity of Milli-Q water per batch was taken in another manufacturing vessel in which weighed quantity of poloxamer-188 was dissolved by heating at 55°C (50°C to 60°C). Dextrose and DOTA were subsequently added and dissolved in the above solution.

### Preparation of NLC:

**High Shear Homogenization:** The lipid phase was added into the aqueous phase, under homogenization at 4000 rpm by maintaining the product temperature at 55°C (50°C to 60°C) and the homogenization was continued further for 15 min at 8500 rpm. Later, the volume was adjusted up to required level with remaining quantity of Milli-Q water followed by homogenization at 8500 rpm for 15 minutes for formation of a coarse dispersion.

**Pressure Homogenization:** The obtained coarse dispersion was subjected to high pressure homogenization for 3 passes at different pressures i.e. Pass 1 at 5,000 psi, Pass 2 at 10,000 psi and Pass 3 at 18,000 psi.

The pH of the formulation is adjusted to 7.0-7.5 with Tris base.

### Example 41: Cannabidiol (CBD) oral NLC formulation

| **S.No** | **Ingredients** | **Quantity (mg/mL)** | **(%w/V)** |
|---|---|---|---|
| 1 | Labrafac Lipophile WL 1349 (Medium Chain Triglycerides NF) | 15 | 1.5 |
| 2 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 6 | 0.6 |
| 3 | cannabidiol synthetic | 15 | 1.5 |
| 4 | ethanol | 10 | 1.0 |
| 5 | egg lecithin (Lipoid E80S ) | 10 | 1.0 |
| 6 | poloxamer-188 | 10 | 1.0 |
| 7 | ascorbyl palmitate | 0.2 | 0.02 |
| 8 | 1,4,7,10-Tetraazacyclododecane-1,4,7,10-tetraacetic acid (DOTA) | 0.02 | 0.002 |
| 9 | propyl paraben | 0.2 | 0.02 |
| 10 | tris base | 0.1 | 0.01 |
| 11 | dextrose anhydrous | 35 | 3.5 |
| 12 | neotame | 1 | 0.1 |
| 13 | peppermint supreme | 2 | 0.2 |
| 14 | menthol | 0.4 | 0.04 |
| 15 | Milli-Q Water | Q.S to 1 mL | Q.S to 100% |

### Manufacturing Procedure:

**Preparation of Oil Phase:** Accurately weighed quantity of Labrafac Lipophile WL 1349 (oil) was taken into a manufacturing vessel. Weighed quantities of PEG-PE and egg lecithin (Lipoid E80S) were subsequently added and dissolved by heating on a water bath at 55°C (50°C to 60°C) to give a mixture which was cooled to 40°C. Weighed amount of CBD was added to the above mixture and dissolved. In another vessel peppermint supreme, propyl paraben, ascorbyl palmitate, neotame and menthol were dissolved in ethanol. The contents of both the vessels were mixed to give a lipid phase.

**Preparation of Aqueous Phase:** 90% of required quantity of Milli-Q water per batch was taken in another manufacturing vessel in which weighed quantity of poloxamer-188 was dissolved by heating at 55°C (50°C to 60°C). Dextrose and DOTA were subsequently added and dissolved in the above solution.

### Preparation of NLC:

**High Shear Homogenization:** The lipid phase was added into the aqueous phase, under homogenization at 4000 rpm by maintaining the product temperature at 55°C (50°C to 60°C) and the homogenization was continued further for 15 min at 8500 rpm. Later, the volume was adjusted up to required level with remaining quantity of Milli-Q water followed by homogenization at 8500 rpm for 15 minutes for formation of a coarse dispersion.

**High Pressure Homogenization:** The obtained coarse dispersion was subjected to high pressure homogenization for 3 passes at different pressures i.e. Pass 1 at 5,000 psi, Pass 2 at 10,000 psi and Pass 3 at 18,000 psi.

The pH of the formulation is adjusted to 7.0-7.5 with tris base.

### Example 42: Cannabidiol (CBD) oral NLC formulation

| **S. No** | **Ingredients** | **Quantity (mg/mL)** | **(%w/V)** |
|---|---|---|---|
| 1 | Labrafac Lipophile WL 1349 (Medium Chain Triglycerides NF) | 20 | 2.0 |
| 2 | N-(Carbonyl-methoxypolyethylenglycol-2000)-1,2-distearoyl-sn-glycero-3-phosphoethanolamine, MPEG-2000-DSPE, Na-salt (Lipoid PE 18:0/18:0-PEG 2000) | 6 | 0.6 |
| 3 | cannabidiol synthetic | 20 | 2.0 |
| 4 | ethanol | 10 | 1.0 |
| 5 | egg lecithin (Lipoid E80S ) | 10 | 1.0 |
| 6 | poloxamer-188 | 10 | 1.0 |
| 7 | ascorbyl palmitate | 0.2 | 0.02 |
| 8 | 1,4,7,10-Tetraazacyclododecane-1,4,7,10-tetraacetic acid (DOTA) | 0.02 | 0.002 |
| 9 | propyl paraben | 0.2 | 0.02 |
| 10 | tris base | 0.1 | 0.01 |
| 11 | dextrose anhydrous | 35 | 3.5 |
| 12 | neotame | 1 | 0.1 |
| 13 | peppermint supreme | 2 | 0.2 |
| 14 | menthol | 0.4 | 0.04 |
| 15 | Milli-Q Water | Q.S to 1 mL | Q.S to 100% |

### Manufacturing Procedure:

**Preparation of Oil Phase:** Accurately weighed quantity of Labrafac Lipophile WL 1349 (oil) was taken into a manufacturing vessel. Weighed quantities of PEG-PE and egg lecithin (Lipoid E80S) were subsequently added and dissolved by heating on a water bath at 55°C (50°C to 60°C) to give a mixture which was cooled to 40°C. Weighed amount of CBD was added to the above mixture and dissolved. In another vessel peppermint supreme, propyl paraben, ascorbyl palmitate, neotame and menthol were dissolved in ethanol. The contents of both the vessels were mixed to give a lipid phase.

**Preparation of Aqueous Phase:** 90% of required quantity of Milli-Q water per batch was taken in another manufacturing vessel in which weighed quantity of poloxamer-188 was dissolved by heating at 55°C (50°C to 60°C). Dextrose and DOTA were subsequently added and dissolved in the above solution.

### Preparation of NLC:

**High Shear Homogenization:** The lipid phase was added into the aqueous phase, under homogenization at 4000 rpm by maintaining the product temperature at 55°C (50°C to 60°C) and the homogenization was continued further for 15 min at 8500 rpm. Later, the volume was adjusted up to required level with remaining quantity of Milli-Q water followed by homogenization at 8500 rpm for 15 minutes for formation of a coarse dispersion.

**High Pressure Homogenization:** The obtained coarse dispersion was subjected to high pressure homogenization for 3 passes at different pressures i.e. Pass 1 at 5,000 psi, Pass 2 at 10,000 psi and Pass 3 at 18,000 psi.

The pH of the formulation is adjusted to 7.0-7.5 with tris base.

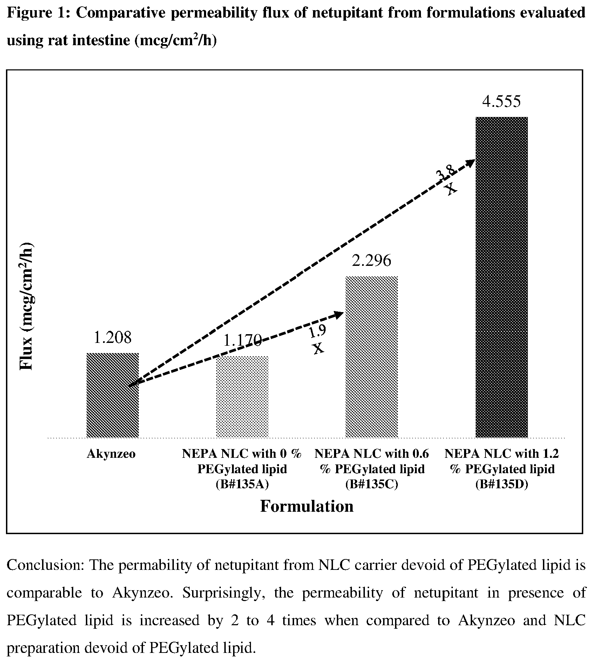

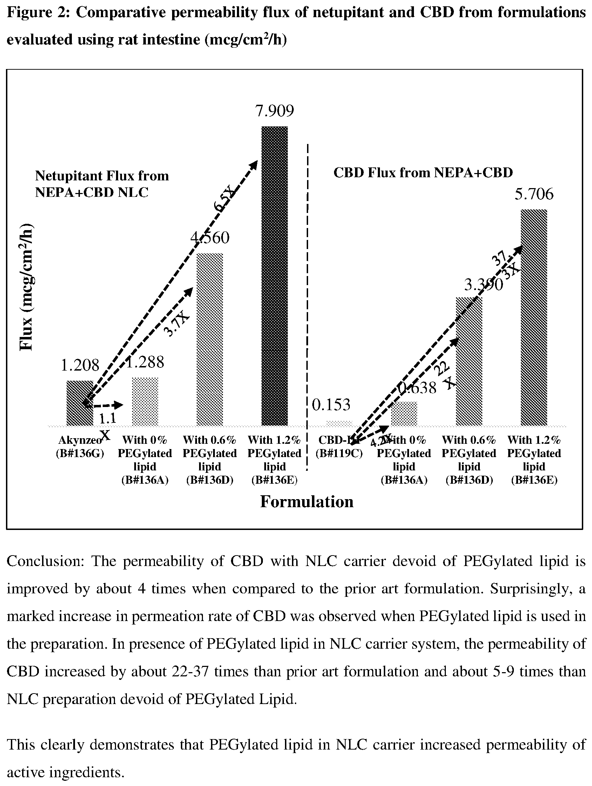

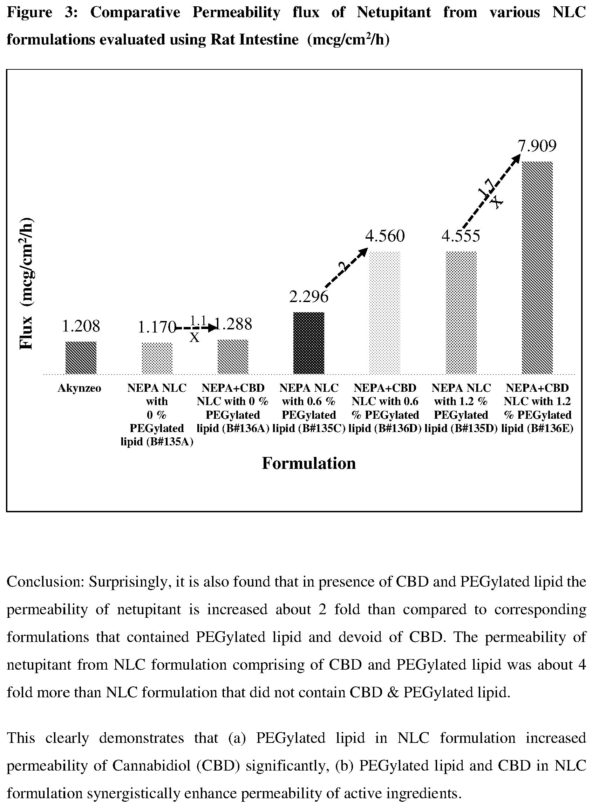

## Claims

1. : A nanolipid carrier system suitable for oral administration, comprising:
i. cannabidiol and one or more low phase transition substances comprising a low phase transition lipid or mixtures thereof,
ii. oil,
iii. PEGylated lipid,
iv. surfactant, and
v. a co-solvent.

2. : The carrier system of claim 1, wherein the permeation rate of cannabidiol is increased by about 2 fold.

3. : The carrier system of claim 1, wherein the hydrodynamic diameter when measured in aqueous dispersion is less than about 1000nm.

4. : The carrier system of claim 1, wherein the hydrodynamic diameter when measured in aqueous dispersion is less than about 700nm.

5. : The carrier system of claim 1, wherein the wherein molecular weight of PEG in the PEGylated lipid ranges from 200Da to 20000 Da.

6. : The carrier system of claim 1, wherein the PEGylated lipid is PEG-PE.

7. : The carrier system of claim 1, wherein the low phase transition lipid used has a phase transition temperature below 80°C.

8. : The carrier system of claim 1, wherein the low phase transition lipid is present upto 20% by weight of the formulation.

9. : The carrier system of claim 1, wherein cannabidiol is present upto 35%by weight of the composition

10. : The carrier system of claim 1, wherein the oil is selected from the group comprising medium chain triglycerides, fish Oil, grape seed oil and soyabean oil.

11. : The carrier system of claim 1, wherein the low phase transition lipid is selected from the group comprising: decanoic acid, Monosteol, alpha lipoic acid and cholesterol.

12. : A nanolipid drug-carrier system suitable for oral administration, comprising:
i. cannabidiol
ii. oil,
iii. PEG-PE,
iv. surfactant, and
v. a co-solvent.

13. : The carrier system of claim 12, wherein the permeation rate of cannabidiol is increased by about 2 fold.

14. : A nanolipid carrier system suitable for oral administration, comprising:
i. Cannabidiol,
ii. One or more active ingredients
iii. oil,
iv. PEGylated lipid,
v. surfactant, and
vi. a co-solvent.

15. : The carrier system of claim 14 where in the one or more active ingredients are selected from the group comprising netupitant, palonosetron, atorvastatin, gabapentin, naproxen, abiraterone, cabazitaxel, paclitaxel, duloxetine and many other drugs and there combinations thereof.

16. : The carrier system of claim 14, wherein the permeability of the active ingredient is increased by about 2 fold.

## Patentansprüche

1. Nanolipidträgersystem, das zur oralen Verabreichung geeignet ist, umfassend:
i. Cannabidiol und einen oder mehrere Stoffe mit niedrigem Phasenübergang, die ein Lipid mit niedrigem Phasenübergang umfassen, oder Gemische davon,
ii. Öl,
iii. PEGyliertes Lipid,
iv. Tensid und
v. ein Colösemittel.

2. Trägersystem nach Anspruch 1, wobei die Permeationsrate von Cannabidiol um das etwa 2-Fache erhöht ist.

3. Trägersystem nach Anspruch 1, wobei der hydrodynamische Durchmesser bei Messung in wässriger Dispersion weniger als etwa 1000 nm beträgt.

4. Trägersystem nach Anspruch 1, wobei der hydrodynamische Durchmesser bei Messung in wässriger Dispersion weniger als etwa 700 nm beträgt.

5. Trägersystem nach Anspruch 1, wobei das Molekulargewicht von PEG in dem PEGylierten Lipid im Bereich von 200 Da bis 20000 Da liegt.

6. Trägersystem nach Anspruch 1, wobei das PEGylierte Lipid PEG-PE ist.

7. Trägersystem nach Anspruch 1, wobei das verwendete Lipid mit niedrigem Phasenübergang eine Phasenübergangstemperatur von unter 80 °C aufweist.

8. Trägersystem nach Anspruch 1, wobei das Lipid mit niedrigem Phasenübergang bis zu 20 Gew.-% der Formulierung ausmacht.

9. Trägersystem nach Anspruch 1, wobei Cannabidiol bis zu 35 Gew.-% der Zusammensetzung ausmacht.

10. Trägersystem nach Anspruch 1, wobei das Öl aus der Gruppe umfassend mittelkettige Triglyceride, Fischöl, Traubenkernöl und Sojaöl ausgewählt ist.

11. Trägersystem nach Anspruch 1, wobei das Lipid mit niedrigem Phasenübergang aus der Gruppe umfassend Decansäure, Monosteol, Alpha-Liponsäure und Cholesterin ausgewählt ist.

12. Nanolipid-Arzneimittelträgersystem, das zur oralen Verabreichung geeignet ist, umfassend:
i. Cannabidiol
ii. Öl,
iii. PEG-PE,
iv. Tensid und
v. ein Colösemittel.

13. Trägersystem nach Anspruch 12, wobei die Permeationsrate von Cannabidiol um das etwa 2-Fache erhöht ist.

14. Nanolipidträgersystem, das zur oralen Verabreichung geeignet ist, umfassend:
i. Cannabidiol,
ii. einen oder mehrere Wirkstoffe
iii. Öl,
iv. PEGyliertes Lipid,
v. Tensid und
vi. ein Colösemittel.

15. Trägersystem nach Anspruch 14, wobei der eine oder die mehreren Wirkstoffe aus der Gruppe umfassend Netupitant, Palonosetron, Atorvastatin, Gabapentin, Naproxen, Abirateron, Cabazitaxel, Paclitaxel, Duloxetin und vielen anderen Arzneimitteln und Kombinationen davon ausgewählt sind.

16. Trägersystem nach Anspruch 14, wobei die Permeabilität des Wirkstoffs um das etwa 2-Fache erhöht ist.

## Revendications

1. Système vecteur de nanoparticules lipidiques pour l'administration orale, comprenant :
i. du cannabidiol et
une ou plusieurs substances à faible transition de phase comprenant un lipide à faible transition de phase ou des mélanges de celles-ci,
ii. de l'huile,
iii. un lipide pégylé,
iv. un tensioactif, et
v. un cosolvant.

2. Système vecteur de la revendication 1, dans lequel le taux de perméation du cannabidiol est multiplié par 2 environ.

3. Système vecteur de la revendication 1, dans lequel le diamètre hydrodynamique, lorsque mesuré dans une dispersion aqueuse, est inférieur à environ 1000 nm.

4. Système vecteur de la revendication 1, dans lequel le diamètre hydrodynamique, lorsque mesuré dans une dispersion aqueuse, est inférieur à environ 700 nm.

5. Système vecteur selon la revendication 1, dans lequel le poids moléculaire du PEG dans le lipide pégylé est compris entre 200 Da et 20 000 Da.

6. Système vecteur de la revendication 1, dans lequel le lipide pégylé est le PEG-PE.

7. Système vecteur de la revendication 1, dans lequel le lipide à faible transition de phase utilisé a une température de transition de phase inférieure à 80°C.

8. Système vecteur de la revendication 1, dans lequel le lipide à faible transition de phase est présent jusqu'à 20% en poids de la formulation.

9. Système vecteur de la revendication 1, dans lequel le cannabidiol est présent jusqu'à 35% en poids de la composition.

10. Système vecteur de la revendication 1, dans lequel l'huile est choisie dans le groupe comprenant les triglycérides à chaîne moyenne, l'huile de poisson, l'huile de pépins de raisin et l'huile de soja.

11. Système vecteur de la revendication 1, dans lequel le lipide à faible transition de phase est choisi dans le groupe comprenant : l'acide décanoïque, le Monosteol, l'acide alpha-lipoïque et le cholestérol.

12. Système vecteur de nanomédicaments lipidiques pour l'administration orale, comprenant :
i. du cannabidiol
ii. de l'huile,
iii. du PEG-PE,
iv. un tensioactif, et
v. un cosolvant.

13. Système vecteur de la revendication 12, dans lequel le taux de perméation du cannabidiol est multiplié par 2 environ.

14. Système vecteur de nanoparticules lipidiques pour l'administration orale, comprenant :
i. du cannabidiol,
ii. un ou plusieurs principes actifs
iii. de l'huile
iv. un lipide pégylé
v. un tensioactif, et
vi. un cosolvant.

15. Système vecteur de la revendication 14, dans lequel le ou les plusieurs principes actifs sont choisis dans le groupe comprenant le nétupitant, le palonosétron, l'atorvastatine, la gabapentine, le naproxène, l'abiratérone, le cabazitaxel, le paclitaxel, la duloxétine et de nombreux autres médicaments ainsi que leurs combinaisons.

16. Système vecteur de la revendication 14, dans lequel la perméabilité du principe actif est multipliée par 2 environ.
